# EUROPEAN PATENT SPECIFICATION

(11) **EP 1 228 770 B1**
(45) Date of publication and mention of the grant of the patent: **13.07.2005**
(21) Application number: 01102223.3
(22) Date of filing: 31.01.2001
(51) Int. Cl.: A61K 49/00

(54) **Lyophilisable contrast agent comprising gas microbubbles**
Lyophilisierbares Kontrastmittel, gasgefüllte Mikrobläschen enthaltend
Agent de contraste lyophilisable comprenant des microbulles de gaz

(43) Date of publication of application: 07.08.2002
(73) Proprietor: BRACCO RESEARCH S.A., 1258 Plan-les-Ouates (CH)
(72) Inventor: Schneider, Michel, 1256 Troinex (CH); Feng, Yan, 1217 Meyrin (CH); Brochot, Jean, 74160 Feigères (FR); Lazarus, David, 74160 Saint-Julien en Genevois (FR)

(56) References cited:
- WO-A-97/29782
- WO-A-99/08716
- US-A- 5 769 080

## Description

### Technical Field

The present invention relates to a process for the preparation of a dried (or dehydrated) contrast agent containing a gas or a gas mixture usable in ultrasonic echography, in nuclear medicine or in magnetic resonance imaging (MRI). The invention also comprises a lyophilised contrast agent obtained by this process as well as a two component kit for the reconstitution of an injectable suspension of air or gas filled microbubbles and their use as contrast agents in diagnostic imaging of human and animal body.

### Background of Invention

Rapid development of ultrasound contrast agents in the recent years has generated a number of different formulations, which are useful in ultrasound imaging of organs and tissue of human or animal body. These agents are designed to be used primarily as intravenous or intra-arterial injectables in conjunction with the use of medical echographic equipment which employs for example, B-mode image formation (based on the spatial distribution of backscatter tissue properties) or Doppler signal processing (based on Continuous Wave or pulsed Doppler processing of ultrasonic echoes to determine blood or liquid flow parameters). Injectable formulations useful as ultrasound contrast agents which comprise suspensions of gas microbubbles in aqueous liquid carriers may basically be divided into two categories. Free gas bubbles are not included in these categories since they are not stable enough. Interest has accordingly been shown in methods of stabilising gas bubbles for echography and other ultrasonic studies, for example using emulsifiers, oils, thickeners or sugars, or by entraining or encapsulating the gas or a precursor therefore in a variety of systems. The first category is aqueous suspensions in which the gas microbubbles are bounded at the gas/liquid interface by a very thin envelope involving the surfactant bound at the gas to liquid interface. The second category is suspensions in which the microbubbles have a solid material envelope formed of natural or synthetic polymers. In the latter case the gas filled particles are referred to as microballoons. There is yet another kind of ultrasound contrast agents: suspensions of porous particles of polymers or other solids, which carry gas microbubbles, entrapped within the pores of the microparticles. These contrast agents are considered here as a variant of the microballoon kind. More on these different formulations may be found in US-A-4,466,442 (EP-A-0 077 752/Schering), EP-A-0 123 235 (Schering), EP-A-0 324 938 (Widder et al.), US-A-5,271,928 (EP-A-0 474 833/Schneider et al.), US-A-5,711,933 (EP-A-0 458 745/Bichon et al.), US-A-4,900,540 (Ryan), US-A-5,230,882 (Unger), etc.

Use of suspensions of gas microbubbles in carrier liquid, as efficient ultrasound reflectors is well known in the art. The development of microbubbles suspensions as echopharmaceuticals for enhancement of ultrasound imaging followed early observations that rapid intravenous injections of aqueous solutions can cause dissolved gases to come out of solution by forming bubbles. Due to their substantial difference in acoustic impedance relative to blood, these intravascular gas bubbles were found to be excellent reflectors of ultrasound. Thus injecting into the blood stream of a living organism suspensions of gas microbubbles in a carrier liquid strongly reinforces ultrasonic echography imaging, thus enhancing the visualisation of internal organs. Since imaging of organs and deep seated tissues can be crucial in establishing medical diagnosis, a lot of effort has been devoted to the development of stable suspensions of highly concentrated gas microbubbles which at the same time would be simple to prepare and administer, would contain a minimum of inactive species and would be capable of long storage and simple distribution. Many attempts towards solutions satisfying these criteria have been made; however, none have provided a completely satisfactory result.

Although the prior art compositions have merit, they still suffer several drawbacks, which hamper their practical use. Firstly, some prior art compositions have relatively short life spans and secondly, they have a relatively low initial bubble count e.g. between 10⁴ and 10⁵ bubbles/ml. This makes reproducibility and analysis of echographic tests made with such compositions fairly difficult. In addition, some techniques produce bubbles in a wide range of diameters (up to 50 µm) which prevents their use as echographic agents in certain applications since echography of the left heart and of the general circulation requires bubbles sizes smaller than 8-10µm.

The need for stable formulations of microbubbles which, will resist pressure variations in the blood stream and have a good shelf life is further amplified by poor stability of some of the state-of-the-art compositions. Microbubbles formulations whose distribution and storage would not present problems are particularly important.

Easy-to-produce aqueous suspensions usable as imaging agents in ultrasonic echography are disclosed in US-A-5,271,928 (Schneider et. al.). The suspensions contain film forming surfactants in laminar and/or lamellar form and, optionally, hydrophilic stabilisers. These microbubbles are stabilised by one or more monomolecular layer(s) of amphipathic compounds i.e. compounds with hydrophilic and hydrophobic moieties. The suspensions are obtained by exposing the laminarized surfactants to air or a gas prior to or after admixing with an aqueous phase. Conversion of film forming surfactants into lamellar form is carried out according to various liposome-forming techniques including high-pressure homogenisation or sonication under acoustic or ultrasonic frequencies. The concentration of phospholipids claimed is between 0.01% and 20% and the concentration of microbubbles is between 10⁸ and 10⁹ bubbles/ml. The above cited patent US-A-5,271,928, also comprises a dry composition which, upon admixing with an aqueous carrier liquid, will generate a sterile suspension of microbubbles thereafter usable as contrast agent for ultrasonic echography. This dry pulverulent composition contains one or more film forming surfactants in laminar form and hydrosoluble stabilizers.

US-A-5,445,813 (EP-B-0 619 743/Schneider et al) is based on the unexpected finding that very stable suspensions of a gas filled microbubbles comprising at least 10⁷ microbubbles per millilitre may be obtained using phospholipids as stabilisers even if very low concentrations thereof are employed. The suspensions usable as contrast agents in ultrasonic echography are obtained by suspending in an aqueous carrier at least one phospholipid as a stabiliser of the microbubbles against collapse with time and pressure, the concentration of the phospholipids being below 0.01% wt. but equal to or higher than that at which the phospholipid molecules are present solely at the gas microbubbles-liquid interface.

US-A-5,413,774 (EP-B-0 554 213/Schneider et al) suggests that one may impart resistance against collapse under pressure to gas-filled microvesicles by introduction thereto of at least one gas whose solubility in water, expressed in liters of gas/liters of water under standard conditions, divided by the square root of its molecular weight does not exceed 0.003. Preferred physiologically acceptable gases are said to include sulphur hexafluoride, various Freon® such as CF₄, CBrF₃, C₄F₈, CClF₃, CCl₂F₂, C₂F₆, C₂ClF₅, CBrClF₂, C₂Cl₂F₄, CBr₂F₂ and C₄F₁₀. Such gases may, inter alia, be used in phospholipid-containing compositions of the type described in the above-mentioned US-A-5,271,928.

US-A-5,556,610 (WO-A-9516467/Yan et al) discloses use of ultrasound contrast media containing a mixture of gases A and B, where gas B is present in an amount of 0.5 - 41% v/v, has a molecular weight greater than 80 daltons and has aqueous solubility below 0.0283 ml/ml water under standard conditions, the balance of the mixture being gas A.
Representative gases A include air, oxygen, nitrogen, carbon dioxide and mixtures thereof. Representative gases B include fluorine-containing gases such as sulphur hexafluoride and various perfluorinated hydrocarbons. Preferred stabilisers in such contrast media include phospholipids.

Storage and transportation of such contrast agents is made significantly more efficient if the microbubbles can be stored in a dried form. The dry form does not require the need of temperature control and special storage facilities for storage and transportation. That's the reason why, the storage of lyophilised contrast agents has shown a particular interest and many attempts have been recently carried out.

For example, it has been suggested by WO 97/29782 (Nycomed) that, a freeze-dried vesicle containing ultrasound contrast agent containing a freeze-drying stabiliser and thermally stable at temperature in excess of 20°C could be obtained. The stabilizer used according to the invention may be a physiologically tolerable freeze-drying stabilizer (or mixture) having a glass transition temperature (Tg) above 20°C and having a Tg' value of -37°C or above. Examples of suitable stabilizers include sucrose, maltose H2O, trehalose, raffinose and stachyose.

One of the purposes of pharmaceutical packaging is to provide adequate product protection. The container forms the primary barrier required for assurance of quality over the shelf life of the product. Certain products, particularly those that are lyophilised, must also be protected from moisture and may need to be protected from oxygen. Contrast agents comprising gas filled microbubbles are particularly storage sensitive. The problems of storage are intrinsic to aqueous gas suspensions, which due to their very nature, undergo phase separation or segregation, gas bubble coalescence, gas diffusion and, after long periods, even precipitation of various additives.
Prior art microbubble-containing contrast agents, are typically prepared by contacting powdered surfactant, e.g. freeze dried or spray-dried phospholipid solutions, with air or other gas and then with aqueous carrier, agitating to generate a microbubble suspension which must then be administered shortly after its preparation. Such processes, however, suffer the disadvantages that substantial agitational energy must be imparted to generate the required dispersion and that the size and size distribution of the microbubbles are dependent on the amount of energy applied and so cannot in practice be controlled.

In addition, convenient containers have to be selected. Storing lyophilised contrast agents at atmospheric pressure also requires very adapted connection systems as well as containers. Reconstitution of lyophilised product with a given volume of aqueous liquid carrier will create an overpressure inside the container. Convenient connection systems or adapters may be disclosed in WO 98/13006 (Biodome) as well as in US 4,787,898 (Raines). The above adapters require the presence of a vent in their structure in order to adjust the container's pressure after reconstitution of the contrast agent. The cited devices for sealing closed containers and adapters are expensive and are not very easy to handle.

Recently it has been disclosed that pharmaceuticals may also been sealed in containers under reduced pressure. Daukas et al. in PDA Journal of Pharmaceutical Science & Technology Vol. 53(1), pp.31-39 (1999) describes comparatives methods for integrity testing of vials sealed at reduced pressures for lyophiles. An advantage of sealing at reduced pressure may lie in promoting ease of reconstitution as the vacuum draws diluent into the vial. Buckley in PDA Journal of Pharmaceutical Science & Technology Vol. 48(4), pp.189-196 (1994) presented the time dependence of pressure in lyophilisation vials. Vials containing lyophilised products are frequently sealed at a nominal chamber pressure Pc < 50 mtorr (1 torr = 1 mm Hg = 1.333 mbar), however vial pressures may also been encountered in the range of 2-100 torr. Controlling the storage atmosphere and facilitating dissolution of solid products before use may also represent other advantages of containers sealed under reduced pressure.

Further difficulties with the storage of dry suspensions can be experienced with ultrasound contrast agents, which contain phospholipids as stabilisers of the gas microbubbles. Such dried products are not conventional lyophiles and therefore have a specific and inherent behaviour. Due to the storage under reduced pressure, the reconstitution of gas filled microbubbles could lead to non-effective contrast agents. Another important problem to be solved is to prevent the alteration of the acoustic properties (echogenic response) after the reconstitution in an aqueous suspension of a dry powder stored for a long time under reduced pressure. In addition, one of the major problem of reconstituted lyophilised microbubbles lies in the presence of very large bubbles (>10 µm). In order to avoid the presence of big bubbles, one object of the present invention is to control the size of the bubbles after reconstitution. Thus so far the problem of storage of ultrasound contrast agents comprising gas microbubbles suspensions remains open.

### Detailed description of the invention

The present invention relates to a process for the preparation of a dried contrast agent containing a gas or a gas mixture usable in diagnostic imaging like ultrasonic echography, magnetic resonance imaging or nuclear medicine. The invention also comprises a lyophilised contrast agent obtained by this process as well as a two component kit for the reconstitution of an injectable suspension of air or gas filled microbubbles and their use as contrast agents in diagnostic imaging of human and animal body. The aim of the invention is to enable extended storage of a dried contrast agent under reduced pressure and still permit reconstitution of stable microbubble suspensions with a reduce percentage of large bubbles.

One aspect of the invention is to provide a contrast agent usable in diagnostic imaging comprising microbubbles of a gas or a gas mixture obtained by reconstitution in a physiologically acceptable liquid aqueous carrier of lyophilisable material obtainable by the process consisting of lyophilising lyophilisable material contained in a container, said lyophilisable material comprising at least one phospholipid film-forming surfactant, effecting sealing closure of said container. Prior to effecting sealing of said container, a vacuum is applied to said container and a reduced pressure of said gas or gas mixture is introduced into said container. The reduced pressure of said gas or gas mixture introduced into said container is comprised between 900 and 100 mbar. Preferably, the reduced pressure of said gas or gas mixture introduced into said container is comprised between 700 and 300 mbar. Even more preferably, the reduced pressure of said gas or gas mixture introduced into said container is equal to 500 mbar. In an alternative embodiment of the invention, the reduced pressure may be introduced into the sealed container just before reconstitution with an acceptable liquid carrier.

In the invention, the reconstituted contrast agent may contain a gas or gas precursor (eg. a compound or mixture of compounds which are substantially in gaseous form (including vapour) at normal human body temperatures (37 °C) i.e. C₅F₁₂, C₆F₁₄ etc..). Any biocompatible gas, gas precursor or mixture may be employed. It is interesting to note that, gases or gas precursors such as perfluorocarbons with a boiling point close to the room temperature (i.e. C₅F₁₂ or C₆F₁₄) may be used in the present invention. One of the advantage of the reduced pressure is to sustain the gas or gas precursor in its vapor state and thus to avoid any condensation of that gas or gas precursor. The gas may thus, for example, comprise air; nitrogen; oxygen; carbon dioxide; hydrogen; nitrous oxide; an inert gas such as helium, argon, xenon or krypton; radioactive gases such as Xe¹³³ or Kr⁸¹; an hyperpolarized gas such as hyperpolarized helium or hyperpolarized noble gases such as hyperpolarized xenon or hyperpolarized neon; a sulphur fluoride such as sulphur hexafluoride or trifluoromethylsulphur pentafluoride; an optionally halogenated silane such as tetramethylsilane; a low molecular weight hydrocarbon (e.g. containing up to 7 carbon atoms), for example an alkane such as methane, ethane, a propane, a butane or a pentane, a cycloalkane such as cyclobutane or cyclopentane, an alkene such as propene or a butene, or an alkyne such as acetylene; an ether; a ketone; an ester; a halogenated low molecular weight hydrocarbon (e.g. containing up to 7 carbon atoms); or a mixture of any of the foregoing. At least some of the halogen atoms in halogenated gases advantageously are fluorine atoms.

Thus biocompatible halogenated hydrocarbon gases may, for example, be selected from bromochlorodifluoromethane, chlorodifluoromethane, dichlorodifluoromethane, bromotrifluoromethane, chlorotrifluoromethane, chloropentafluoroethane, dichlorotetrafluoroethane and perfluorocarbons, e.g. perfluoroalkanes such as perfluoromethane, perfluoroethane, perfluoropropanes, perfluorobutanes (e.g. perfluoro-n-butane, optionally in admixture with other isomers such as perfluoro-isobutane), perfluoropentanes, perfluorohexanes and perfluoroheptanes: perfluoroalkenes such as perfluoropropene, perfluorobutenes (e.g. perfluorobut-2ene) and perfluorobutadiene; perfluoroalkynes such as perfluorobut-2-yne; and perfluorocycloalkanes such as perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane and perfluorocycloheptane. Other halogenated gases include fluorinated, e.g. perfluorinated, ketones such as perfluoroacetone and fluorinated, e.g. perfluorinated, ethers such as perfluorodiethyl ether.

For ultrasonic echography, the biocompatible gas or gas mixture may be preferably selected from the group consisting of air, nitrogen, carbon dioxide, helium, krypton, xenon, argon, methane, hyperpolarized gases, halogenated hydrocarbons (including fluorinated gases such as perfluorocarbons), sulphur hexafluoride and mixtures thereof. The hyperpolarized gases may preferably be selected from hyperpolarized helium or hyperpolarized xenon or hyperpolarized neon and mixtures thereof. The halogenated hydrocarbons may preferably consist of perfluorocarbons selected from the group consisting of perfluoromethane, perfluoroethane, perfluoropropane, perfluorobutane, perfluorocyclobutane, perfluoropentane, perfluorohexane and mixtures thereof. For example, the physiologically acceptable gas used may be selected from SF₆, Freon® such as CF₄, CBrF₃, C₄F₈, CClF₃, CCl₂F₂, C₂F₆, C₂ClF₅, CBrClF₂, CBr₂F₂, C₃F₈ and C₄F₁₀ or mixtures thereof.

Particularly preferable contrast agent usable in MRI will contain hyperpolarized neon, hyperpolarized helium, hyperpolarized xenon, or mixtures thereof with air, oxygen, nitrogen, helium, sulphur hexafluoride, perfluoropropane, perfluorobutane or CO₂.

For nuclear medicine, contrast agent according to the invention will preferably contain radioactive gases such as Xe¹³³ or Kr⁸¹ or mixtures thereof with air, oxygen, nitrogen, helium, perfluorocarbons or CO₂.

It has been found that the surfactants, which are convenient in this invention, can be selected from amphipathic compounds capable of forming stable films in the presence of water and gases. The film or membrane may be formed of any phospholipid film-forming material, particularly non-cross-linked phospholipids. Preferably, the phospholipid film forming material may be selected from saturated phospholipids or synthetic non-saturated phospholipids or a mixture thereof. The preferred surfactants include the lecithins (phosphatidylcholine) and other phospholipids, inter alia phosphatidic acid (PA), phosphatidyl-inositol (PI), phosphatidylethanolamine (PE), phosphatidyl-serine (PS), phosphatidylglycerol (PG), cardiolipin (CL), sphingomyelins and mixtures thereof. Examples of suitable phospholipids are natural or synthetic lecithins, such as egg or soya bean lecithin, or saturated synthetic lecithins, such as, dimyristoylphosphatidylcholine, dipalmitoylphosphatidylcholine, distearoylphosphatidylcholine, diarachidoylphosphatidylcholine, dimyristoylphosphatidyl-serine, dipalmitoylphosphatidyl-serine, distearoylphosphatidyl-serine, diarachidoylphosphatidyl-serine, dimyristoylphosphatidylglycerol, dipalmitoylphosphatidylglycerol, distearoylphosphatidylgycerol, diarachidoylphosphatidylglycerol, dimyristoylphosphatidylethanolamine, dipalmitoylphosphatidylethanolamine, distearoylphosphatidylethanolamine, diarachidoylphosphatidylethanolamine, dimyristoylphosphatidic acid, dipalmitoylphosphatididic acid, distearoylphosphatididic acid, diarachidoylphosphatididic acid, dimyristoylphosphatidyl-inositol, dipalmitoylphosphatidyl-inositol, distearoylphosphatidyl-inositol or diarachidoylphosphatidyl-inositol or unsaturated synthetic lecithins, such as dioleylphosphatidyl choline or dilinoleylphosphatidylcholine or mixed chains phosphatidylcholines such as for instance monooleyl-monopalmitoylphosphatidylcholine, with saturated lecithins being preferred.

In a preferred embodiment of the invention, the phospholipid film-forming surfactant may be a natural or synthetic saturated or a non-saturated phospholipid or mixtures thereof. Preferably, the phospholipid film-forming surfactant may be a saturated phospholipid or a synthetic non-saturated phospholipid. Even more preferably, the saturated phospholipid may be selected from saturated phosphatidic acid, saturated phosphatidylcholine, saturated phosphatidyl-ethanolamine, saturated phosphatidyl-serine, saturated phosphatidylglycerol, saturated phosphatidyl-inositol, cardiolipin, sphingomyelin and mixtures thereof.

Additives like cholesterol and other substances can optionally be added to one or more of the foregoing lipids in proportions ranging from zero to 50% by weight. Such additives may include other non-phospholipid surfactants that can be used in admixture with the film forming surfactants and most of which are known. An explanation of such additives or blood residence prolonging agents may reside in the fact that they may act as opsonisation inhibitors delaying the uptake of the microbubbles from the vasculature by the reticuloendothelial system. For instance compounds like polyoxypropylene glycol and polyoxyethylene glycol as well as copolymers thereof, ergosterol, phytosterol, sitosterol, lanosterol, tocopherol, propyl gallate, ascorbyl palmitate and butylated hydroxytoluene. The amount of these non-film-forming surfactants is usually up to 50% by weight of the total amount of surfactants but preferably between 0 and 30%. This means that the concentration of the various additives in the phospholipid content suspensions of the invention may be in the range of 0-0.05%.

Preferable additives are selected among dicetylphosphate, cholesterol, ergosterol, phytosterol, sitosterol, lanosterol, tocopherol, propyl gallate, ascorbyl palmitate and butylated hydroxy-toluene. Said contrast agent may further contain viscosity enhancers or stabilizers selected from linear and cross-linked poly- and oligo-saccharides, sugars, hydrophilic polymers like polyoxypropylene glycol and polyoxyethylene glycol. The above contrast agent may further comprise up to 50% by weight of non phospholipid surfactants selected from fatty acids, esters and ethers of fatty acids and alcohols with polyols.

Other excipients may if desired be present in the composition being dried or may be added on formulation for administration. Such excipients may for example include pH regulators, osmolality adjusters, viscosity enhancers, emulsifiers, bulking agents, etc. and may be used in conventional amounts. Since preparation of the contrast agents typically involves a freeze drying or spray drying step as discussed it may, however, be advantageous to include one or more agents with cryoprotective and/or lyoprotective effect and/or one or more bulking agents, for example an amino-acid such as glycine; a carbohydrate, e.g. a sugar such as sucrose, mannitol, trehalose, glucose, lactose or a cyclodextrin, or a polysaccharide such as dextran; or a polyglycol such as polyethylene glycol. A substantial list of agents with cryoprotective and/or lyoprotective effects is given in Acta Pharm. Technol. 34(3), pp. 129-139 (1988), the contents of which are incorporated herein by reference.

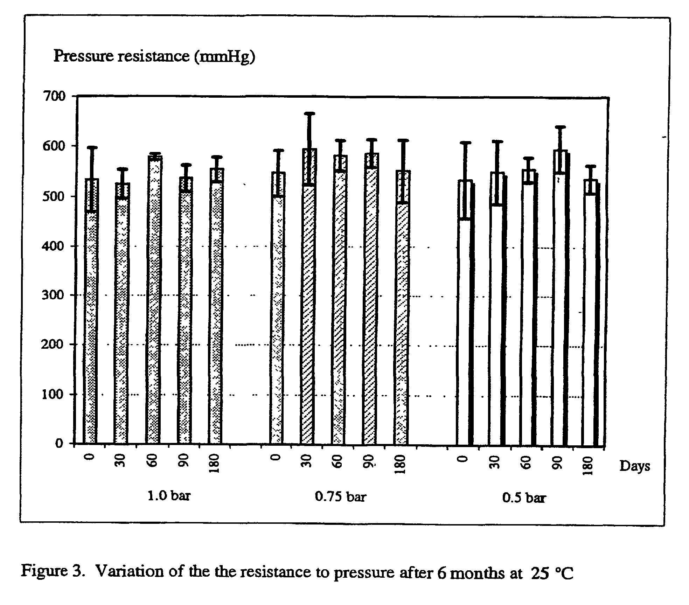

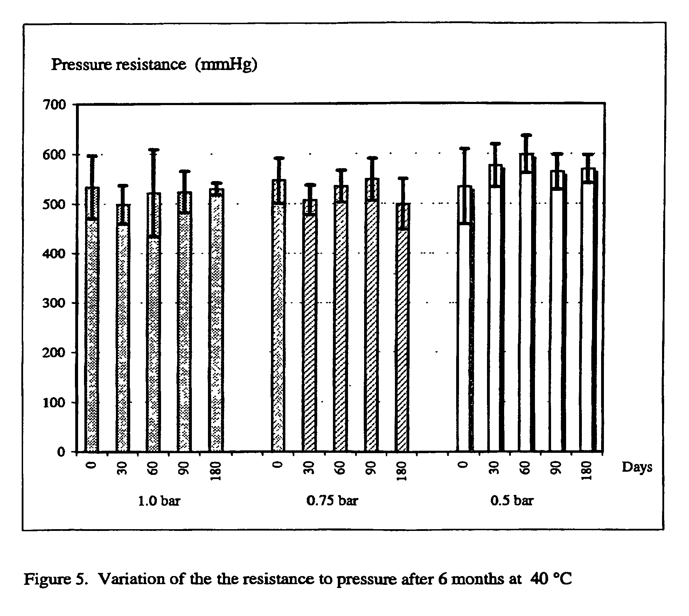

An advantage of the present invention, is that the pressure resistance of the bubbles obtained after the reconstitution of the lyophilised material in an aqueous suspension is retained even after the lyophilised material has been stored in a container under reduced pressure at a temperature of 25°C or 30°C or even 40°C during a period of one month, 2 months, 3 months or even 6 months or more. This characteristic is illustrated in figures 3 and 5. The resistance to pressure (definition given i.e. in E-B-0 554 213) is retained whatever the storage conditions. Surprisingly, it has also been shown that the microbubble properties (bubble concentration, mean size and gas encapsulated volume) remain substantially the same after reconstituting lyophilised products stored under reduced pressure. For example, Table 1 shows that with a reduced pressure of 500mbar, the gas microbubble properties are still fairly good compared to the sample prepared under atmospheric pressure. However, depending on the technique which is used for the microbubbles preparation, we may have little variations in the microbubble properties. Nevertheless, a percentage, which is higher or equal to 10% of the initial bubble concentration, is still sufficient for having a good echogenicity.

It should also be mentioned that another feature of the injectable reconstituted suspensions of the invention is a relatively "high" gas entrapping capacity of the microbubbles i.e. high ratio between the total amount of the entrapped gas and the amount of the surfactant.

Viewed from a further aspect, the invention provides a lyophilised contrast agent usable in diagnostic imaging containing a gas or a gas mixture obtainable by the process consisting of lyophilising lyophilisable material contained in a container, said lyophilisable material comprising at least one phospholipid film-forming surfactant, effecting sealing closure of said container. Prior to effecting sealing of said container, a vacuum is applied to said container and a reduced pressure of said gas or gas mixture is introduced into said container. The reduced pressure of said gas or gas mixture introduced into said container is comprised between 900 and 100 mbar. Preferably, the reduced pressure of said gas or gas mixture introduced into said container is comprised between 700 and 300 mbar. Even more preferably, the reduced pressure of said gas or gas mixture introduced into said container is equal to 500 mbar. The gas or gas mixtures, the phospholipid film forming surfactants, the additives, the viscosity enhancers or stabilizers and the non-phospholipid surfactants useful in the manufacture of such lyophilised contrast agent are already discussed above.

It has been surprisingly found that the lyophilised or dried contrast agent obtained according to the above process has shown improved stability after very long time storage (more than one year).

Conveniently, the lyophilised contrast agent of the invention can be prepared with non-laminarized phospholipids or either with phospholipids, which were lamellarized, or laminarized prior to their contacting with air or another gas. The term lamellar or lamella or laminar form indicates that the surfactants are in the form of thin films or sheets involving one or more monomolecular layer(s). As described in WO-A-91/15244 conversion of film-forming surfactants into lamellar form can easily be done by any liposome forming method for instance by high-speed mechanical homogenisation or by sonication under acoustical or ultrasonic frequencies.

Furthermore, it has also been surprisingly found that by appropriate choice of the reduced pressure, it is possible to produce lyophilised ultrasound or MRI or nuclear medicine contrast agents which are timely stable at ambient temperatures and above, and indeed at all temperatures normally encountered during transportation and storage.

The stable lyophilised contrast agents of the invention may then be stored and transported without need of temperature control of its environment and in particular may be supplied to hospitals and physicians for on site formulation into a ready to-use administrable suspension without requiring such users to have special storage facilities. Lyophilized products according to the invention have proved to be storage stable for several months under ambient conditions. The microbubble dispersions or suspensions generated upon reconstitution in water (or other reconstitution liquids) may be stable for considerable lengths of time, e.g. up to at least 12 hours, permitting considerable flexibility as to when the dried product is reconstituted prior to injection.

A further advantage of the present invention due to the fact that because the dried contrast agent is sealed at reduced pressure it is more easily reconstituted because the vacuum draws diluent into the vial. Sealing under reduced pressure also permits the storage atmosphere to be controlled and facilitates dissolution of lyophilised solid products before use. In an alternative embodiment of the invention, the reduced pressure may be introduced into the sealed container just before reconstitution with the liquid carrier. In the latter embodiment, the lyophilised contrast agent may be stored in a container under atmospheric pressure.

Viewed from a further aspect, the invention provides a process for the preparation of a dried contrast agent containing gas or a gas mixture usable in diagnostic imaging which comprises lyophilising lyophilisable material contained in a container, said lyophilisable material comprising at least one phospholipid film forming surfactant, effecting sealing closure of said container. Prior to effecting sealing of said container, a vacuum is applied to said container and a reduced pressure of said gas or gas mixture is introduced into said container. The reduced pressure of said gas or gas mixture introduced into said container is comprised between 900 and 100 mbar. Preferably, the reduced pressure of said gas or gas mixture introduced into said container is comprised between 700 and 300 mbar. Even more preferably, the reduced pressure of said gas or gas mixture introduced into said container is 500 mbar.

In an alternative embodiment of the invention, the reduced pressure may be applied just before reconstitution of the contrast agent with a physiologically acceptable liquid carrier. The reduced pressure may be introduced into the sealed container just after applying the vacuum to said container.

Viewed from a still further aspect, the invention provides a process for the preparation of an injectable reconstituted suspension of air or gas filled microbubbles usable as imaging contrast agent in diagnostic imaging. The lyophilised contrast agent as defined above is admixed with water or a physiologically acceptable aqueous carrier liquid.

Viewed from a yet still further aspect, the invention provides the use of the lyophilised contrast agent as defined above in a physiologically or pharmaceutically acceptable aqueous liquid carrier for the manufacture of an injectable aqueous suspension of gas filled microbubbles for use in diagnosis involving diagnostic ultrasound imaging or magnetic resonance (MRI) or nuclear medicine.

Viewed from a further aspect, the invention provides a contrast agent for use in diagnostic studies, comprising ultrasonic echography, nuclear medicine or MRI.

Viewed from a still further aspect, the invention provides a method of diagnostic imaging which comprises administering to a subject a contrast-enhancing amount of a contrast agent according to the above description and imaging at least a part of said subject by ultrasound or magnetic resonance or scintigraphy. According to this method, said subject is a vertebrate and said contrast agent is introduced into the vasculature or into a body cavity of said vertebrate.

Viewed from a yet still further aspect, the invention provides a two component kit comprising, as the first component, a lyophilised contrast agent usable in diagnostic imaging containing a gas or a gas mixture as defined above and as the second component a physiologically acceptable carrier liquid which, when admixed with the first component, provides an injectable contrast agent.

Thus viewed from one aspect the invention provides a lyophilised contrast agent containing one or more phospholipid film-forming surfactants which, upon dissolution in water or in an aqueous carrier liquid, will form a injectable aqueous suspension of gas filled microbubbles usable as imaging contrast agent in diagnostic imaging. It is a feature of the invention to prevent totally or significantly the alteration of the acoustic properties (echogenic response) during storage. In adjusting carefully the range of the reduced pressures applied to the container, the acoustic properties will not be affected by storage of the lyophilised powder for extended periods. Thus, one advantage of the present invention is to prevent the alteration of the acoustic properties after the reconstitution in an aqueous suspension of a lyophilised material which has been stored in a container under reduced pressure at a temperature of 20°C or at 30°C or even at 40°C during a period of one month or even two months or more.

For ultrasound applications such as echocardiography, in order to permit free passage through the pulmonary system and to achieve resonance with the preferred imaging frequencies of about 0.1-15 MHz, microbubbles having an average size of 0.1-10 µm are necessary. Contrast agents according to the present invention may be produced with a very narrow size distribution within the range preferred for echocardiography, thereby greatly enhancing their effective echogenicity as well as their safety in vivo, and rendering the contrast agents of particular advantage in applications such as blood pressure measurements, blood flow tracing and ultrasound tomography.
In the present invention, the size of the microbubbles generated is consistently reproducible and in practice is independent of the amount of agitational energy applied, this size parameter surprisingly being substantially maintained in the lyophilised and reconstituted product. Thus, since the size of the microbubbles in the initial dispersion may readily be controlled by process parameters such as the method, the storage under a defined range of reduced pressure, speed and duration of agitation, the final microbubble size may readily be controlled. This avoidance of unlimited size increases which could lead to undesirable and potentially highly dangerous blocking of blood vessel capillaries is a major advantage of such contrast agents. It is shown a pronounced decrease in volume of bubbles larger than 10 µm while bubbles in the range of 2 to 8 µm are less modified by partial pressure. In Example 10, the results show that more than 50% of the large bubbles (>8µm) were eliminated by using a reduced pressure of 500mbar when compared with the sample prepared at atmospheric gas pressure (1000mbar).

Freeze-drying and spray drying techniques can be used to obtain the dried contrast agent of the present invention. The dried or lyophilised product will generally be in powder or in a cake form and is readily reconstitutable in a suitable aqueous liquid carrier, which is physiologically (or pharmaceutically) acceptable, sterile and injectable. Suitable liquid carriers are water, aqueous solutions such as saline (which may advantageously be balanced so that the final product for injection is not hypotonic), or solutions of one or more tonicity adjusting substances such as salts or sugars, sugar alcohols, glycols and other non-ionic polyol materials (eg. glucose, sucrose, sorbitol, mannitol, glycerol, polyethylene glycols, propylene glycols and the like). In practice, all injectable compositions after reconstitution of the lyophilised contrast agent should also be as far as possible isotonic with blood. Hence, before injection, small amounts of isotonic agents may also be added to the suspensions of the invention. The isotonic agents are physiological solutions commonly used in medicine and they comprise aqueous saline solution (0.9% NaCl), 2,6% glycerol solution, 5% dextrose solution, etc. Reconstitution will generally require only minimal agitation such as may, for example, be provided by gentle hand shaking. The size of the microbubbles so generated is consistently reproducible and in practice is independent of the amount of agitational energy applied. Thus, the reconstitution of aqueous microbubbles suspensions is obtained by simple dissolution of the gas-stored dried film forming phospholipids without any violent agitation.

The volume and concentrations of the reconstitution liquid may desirably be balanced to make the resulting ready-to-use formulations substantially isotonic. Hence the volume and concentration of reconstitution fluid chosen will be dependent on the type and amount of stabilizer (and other bulking agents) present in the freeze-dried product.

The bubble suspensions of the present invention are also useful in other medical/diagnostic applications where it is desirable to target the stabilized microbubbles to specific sites in the body following their injection, for instance to thrombi present in blood vessels, to atherosclerotic lesions (plaques) in arteries, to tumor cells, as well as for the diagnosis of altered surfaces of body cavities, e.g. ulceration sites in the stomach or tumors of the bladder. For this, one can bind monoclonal antibodies tailored by genetic engineering, antibody fragments, peptides, oligopeptides or polypeptides designed to mimic antibodies, bioadhesive polymers, lectins and other site-recognizing molecules (ligands) to the phospholipid film-forming surfactant stabilizing the microbubbles. Thus phospholipid derivatives may be obtained i.e. DPPE coupled with N-biotinyl in example 2.

A further advantage of the present invention is that because the gas or gas mixture is introduced into the vial containing the lyophilised product under reduced pressure, less gas must be used. This is a significant advantage because gases such as perfluorocarbons are very expensive. Indeed, applying a reduced pressure in the lyophilisation chamber may reduce the amount of gas from 50 to 80%. For example, using a pressure of 200 mbar allows an economy of 80% of the gas quantity as compared to a contrast agent prepared at atmospheric pressure. This results also in an ecological improvement since less perfluorocarbon gases are released in the atmosphere. Moreover, in the case of very expensive gases such as the hyperpolarized helium, xenon or neon used for magnetic resonance imaging (MRI), the saving may be significant.

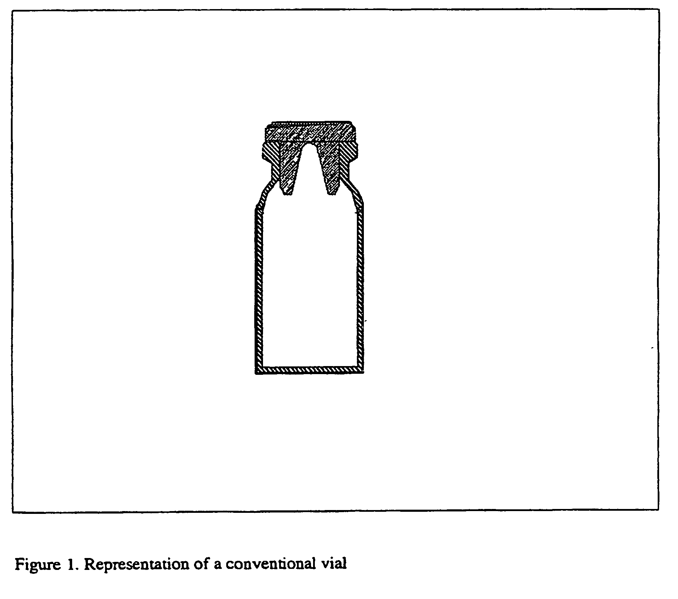

Suitable containers, vials or bottles according to the present invention are for example illustrated in figure 1. No specific vial or connection systems are required; the present invention may use conventional vials and adapters. The only requirement is a good seal between the stopper and the container. The quality of the seal, therefore, becomes a matter of primary concern; any degradation of seal integrity could allow undesirables substances to enter the vial. In addition to assuring sterility, vacuum retention is essential for products stoppered at reduced pressures to assure safe and proper reconstitution. As to the stopper, it may be a compound or multicomponent formulation based on an elastomer, such as poly (isobutylene) or "butyl rubber".

It will be appreciated that kits as illustrated in Example 13, can be prepared for use in making the microbubbles preparations of the present invention. These kits can include a container containing all of the sterile lyophilised material of the present invention and enclosing the gas or gas mixtures described above, in one chamber under reduced pressure. The sterile aqueous liquid may be contained in a second chamber of the same container. In one embodiment, the container is a conventional septum-sealed vial. In another, it has a means for directing or permitting application of sufficient bubble forming energy into the contents of the container. Where the dried product is contained in a vial this is conveniently sealed with a septum through which the carrier liquid may be injected using an optionally prefilled syringe. Alternatively, the dried product and carrier liquid may be supplied together in a two component kit such as a dual chamber syringe. It may be advantageous to mix or gently shake the product following reconstitution. However, as noted above, in the stabilised contrast agents according to the invention the size of the gas microbubbles may be substantially independent of the amount of agitational energy applied to the reconstituted dried product. Accordingly no more than gentle hand shaking may be required to give reproducible products with consistent microbubble size.

The invention has been described above with reference to microbubbles ultrasound contrast agents. However it is also applicable to contrast agents for other diagnostic imaging modalities (eg. MRI, X-ray, SPECT, PET, scintigraphic imaging, magnetic imaging etc.) or even for therapeutically applications.

### Brief description of the drawings

FIG. 1 is an example of a suitable container for the storage of the lyophilised contrast medium according to the invention.
FIG. 2 represents the variation in number of the bubble concentration at 1, 0.75 and 0.5 bar performed at time zero and after 1 month, two months, 3 months and 6 months at 25°C.
FIG. 3 represents the variation of the resistance to pressure in mmHg at 1, 0.75 and 0.5 bar performed at time zero and after 1 month, two months, 3 months and 6 months at 25°C.
FIG. 4 illustrates the variation in number of the bubble concentration at 1, 0.75 and 0.5 bar performed at time zero and after 1 month, two months, 3 months and 6 months at 40°C.
FIG. 5 represents the variation of the resistance to pressure in mmHg at 1, 0.75 and 0.5 bar performed at time zero and after 1 month, two months, 3 months and 6 months at 40°C.

The disclosure of all the above-described patents is incorporated herein by reference.

The foregoing description will be more fully understood with reference to the following Examples. Such Examples, are, however, exemplary of methods of practising the present invention and are not intended to limit the scope of the invention.

### Examples

For lipid components, following abbreviations are used in the description of the Examples:
- DAPC: diarachidoyl phosphatidyl choline
- DCP: dicetyl phosphate
- DPPC: dipalmitoyl phosphatidyl choline
- DPPG: dipalmitoyl phosphatidyl glycerol
- DPPA: dipalmitoyl phosphatidic acid
- DPPS: dipalmitoyl phosphatidyl serine
- DSPC: distearoyl phosphatidyl choline
- DSPS: distearoyl phosphatidyl serine
- DSPE-PEG2000: distearoyl phosphoethanolamine-Npoly(ethylene glycol)2000
- DPPE-PEG5000: dipalmitoyl phosphoethanolamine-Npoly(ethylene glycol)5000
- N-Biotinyl Cap-PE: n-biotinyl caproyl phosphatidylethanolamine
- TAP: distearoyl trimethylammonium propane

All these phospholipids are purchased from Lipoid (Switzerland) and/or from Avanti Polar Lipids (USA).

### Example 1

A solution containing 0.9g of DPPC and 100mg of DPPG was prepared with 50ml of hexane/isopropanol 8/2 v/v; (Fluka, Switzerland). The solvents were evaporated to dryness. 20ml of distilled water were added and the mixture was heated at 65°C for one hour on a rotavapor apparatus. The resulting suspension was then extruded through successively 3 and 1 µm polycarbonate membranes (Nuclepore®). After cooling, the extruded suspension was mixed with Macrogol 4000 solution (100mg/ml; Macrogol 4000 is poly (ethylene glycol) with a molecular weight of 4000 and purchased from Clarian, Germany) with a volume ratio of 1:9 (Lipid suspension/Macrogol solution) and rapidly frozen at -45°C in a round glass flask. The frozen sample was then freeze-dried under vacuum (0.2mbar) and overnight. Aliquots of the obtained lyophilisate were placed in 10ml glass vials (450mg/vial) and sealed with a gas tight stopper. The vials were totally evacuated by high vacuum pump and then filled with C₄F₁₀ gas under different absolute gas pressures (100, 200, 300, 500 and 1000 mbar). The lyophilisate samples were then reconstituted with 5ml saline solution (injected through the stopper) by vigorous shaking on a vortex mixer to generate gas microbubbles. Coulter counter measurements (Multisizer II) were performed on the bubble suspensions to evaluate the bubble characteristics (size and number) under different reduced gas pressures. The results of Coulter measurements are summarized below. Dn corresponds to the mean diameter in number. The bubble volume is the total volume of gas entrapped in the bubbles per ml of reconstituted solution.

**Table 1**

| Pressure (mbar) | Total bubble cone. (10⁹/ml) | Dn (µm) | Bubble volume (µl/ml) |
|---|---|---|---|
| 1000 | 1.50 | 1.8 | 20.0 |
| 900 | 1.30 | 2.0 | 20.5 |
| **500** | **0.87** | **2.0** | **22.1** |
| 300 | 0.41 | 2.2 | 13.6 |
| 200 | 0.42 | 2.2 | 11.6 |
| 100 | 0.23 | 2.4 | 6.0 |

These results surprisingly show that with a reduced pressure of 500mbar, gas microbubbles properties are still fairly good as compared to the sample prepared under normal atmosphere pressure (1000mbar). Even at a pressure as low as 100 mbar, more than 2x10⁸ bubbles/ml are obtained.

### Example 2

A series of aqueous phospholipid suspensions were prepared with the following lipid compositions:
A. 25mg of DAPC, 70mg of DSPS and 5mg of DSPE-PEG2000
B. 45mg of DSPC, 45mg of DPPG, 10mg of palmitic acid
C. 54mg DPPC, 6mg DPPA, 40mg PE-PEG5000
D. 100mg of DPPS
E. 90mg of DPPS, 10mg of DSPE-PEG2000
F. 50mgDPPC, 45mg DPPS, 5mg Pluronic® F108
G. 50mg of DPPG, 150mg of Pluronic® F68, 2.5mg of N-biotinyl Cap-PE
H. 90mg DSPC, 10mg TAP

The components of each composition were dispersed in 50ml aqueous solution containing 1.5g of glycerol and 5g of propylene glycol. All dispersions were heated 1 hour at 75°C on a rotavapor apparatus for lipid hydration. After cooling, the suspensions were then homogenized under high-speed mechanical agitation using a Polytron® (Kinematica AG, 15' 000 rpm and 2min.) at 5°C and under C₄F₁₀ gas in air. The generated bubble suspensions were introduced in 50ml syringes and decanted for one night. The syringes were kept horizontally. After decantation (a white opaque thin bubble layer forms on the top of the solution), the sub-phase (∼45ml) was discarded, the bubble-containing phase (∼5 ml) was recovered and resuspended in the same volume of a 5% Dextran-15 (Mw15000) aqueous solution. This bubble decantation process allows to eliminate the excess lipids not associated with bubbles and thus produce bubble suspensions containing very low phospholipid concentrations (see US patent 5,445,813 incorporated therein by reference in its entirety). The resulting bubble suspensions were, introduced in 10ml glass vials (1 ml per vial) then rapidly frozen at -45°C (see US 5,961,956 incorporated herein by reference) and freeze-dried. After lyophilisation, the vials were evacuated by high vacuum pump and then filled with C₄F₁₀ gas, either under normal (1000 mbar) or reduced gas pressure (500mbar) and sealed with gas tight stoppers. Microbubble suspensions were obtained by injecting through the stopper 0.9% NaCl (5 ml) to each vial followed by vigorous agitation. Coulter counter measurements were performed on all bubble suspensions. The results are summarized below:

**Table 2**

| Samples | total bubble conc. (10⁸/ml) | | Number mean diameter Dn (µm) | | Total bubble volume (µl/ml) | |
|---|---|---|---|---|---|---|
| | (1000mbar) | (500mbar) | (1000mbar) | (500mbar) | (1000mbar) | (500mbar) |
| A | 7.1 | 7.0 | 2.2 | 2.1 | 8.6 | 8.1 |
| B | 2.7 | 2.5 | 2.0 | 2.0 | 7.7 | 8.0 |
| C | 2.1 | 1.5 | 2.4 | 2.3 | 4.5 | 2.8 |
| D | 6.6 | 8.6 | 2.0 | 2.1 | 5.6 | 6.9 |
| E | 4.3 | 4.3 | 2.4 | 2.3 | 10.0 | 9.7 |
| F | 2.7 | 2.5 | 2.1 | 2.2 | 7.5 | 6.9 |
| G | 2.0 | 1.8 | 2.0 | 2.0 | 5.5 | 5.3 |
| H | 0.3 | 0.3 | 2.3 | 2.3 | 3.4 | 3.1 |

These results show that even under half the normal gas pressure (500mbar vs. 1000mbar), the tested formulations maintain the properties of the microbubbles (bubble concentrations, mean sizes and gas encapsulated volumes).

### Example 3

A phospholipid mixture containing 27mg of DPPC, 3mg of DPPA and 20mg of DPPE-PEG5000 was dissolved in 18g of tert-butanol under reflux (82°C). After dissolution, 3g of Macrogol 4000 were added. After complete dissolution, aliquots of the solution were filled into 10ml glass vials (310µl/vial), frozen at -45°C and lyophilized. The lyophilisate-containing vials were evacuated by high vacuum pump, filled with various gases (see below) under different absolute gas pressures (100, 300, 500, 700 and 1000 mbar) and sealed with gas tight stoppers. The lyophilisate samples were reconstituted with 5ml saline solution (injected through the stopper) by vigorous shaking to generate gas microbubbles. Coulter counter analyses were performed and the results are presented in Table 3. The concentration of gas microbubbles prepared at 300, 500 and 700 mbar are expressed as relative bubble concentrations, normalized with the values obtained from samples prepared at 1000mbar (atmospheric pressure).

**Table 3**

| **Bubble conc. (%)** | | | | | |
|---|---|---|---|---|---|
| Gases | *(1000mbar)* | *(700mbar)* | *(500mbar)* | *(300mbar)* | *(100mbar)* |
| Air | 100% (1.3 x 10⁸/ml) | 68% | 23% | 11% | 0% |
| SF6 | 100% (3.7 x 10⁸/ml) | 83% | 45% | 17% | 3% |
| C2F6 | 100% (3.2 x 10⁸/ml) | 96% | 80% | 56% | 16% |
| C4F10 | 100% (3.8 x 10⁸/ml) | 93% | 79% | 70% | 27% |
| C2F6/C4F10 | 100% (4.0 x 10⁸/ml) | 76% | 66% | 52% | 27% |
| (90:10) | | | | | |
| C4F10/air | 100% (3.2 x 10⁸/ml) | 85% | 73% | 59% | 25% |
| (35:65) | | | | | |
| C3F8/air | 100% (3.6 x 10⁸/ml) | 104% | 79% | 63% | 31% |
| (65:35) | | | | | |

The nature of the gas or gas mixture appears to have an important effect on the microbubble concentration under reduced gas pressure, especially at the low-pressure values (100-500mbar). It should be noted that it is not mandatory to achieve the same bubble concentration at reduced pressure compared to atmospheric pressure. A product containing 10⁷ bubbles/ml can be useful as echographic contrast agent.

### Example 4

Lyophilisates were prepared as described in Example 3 except for the phospholipid composition: 47.5mg of DSPC, 2.5mg of DCP were used here (instead of DPPC/ DPPA/DPPE-PEG5000). The Coulter counter results are given in Table 4.

**Table 4**

| **Bubble conc. (%)** | | | | | |
|---|---|---|---|---|---|
| Gases | *(1000mbar)* | *(700mbar)* | *(500mbar)* | *(300mbar)* | *(100mbar)* |
| Air | 100% (1.2 x 10⁸/ml) | 31% | 21% | 9% | 0% |
| SF6 | 100% (3.0 x 10⁸/ml) | 76% | 50% | 26% | 2% |
| C2F6 | 100% (3.5 x 10⁸/ml) | 58% | 56% | 43% | 8% |
| C4F10 | 100% (3.9 x 10⁸/ml) | 74% | 54% | 53% | 12% |
| C2F6/C4F10 (90:10) | 100% (3.5 x 10⁸/ml) | 73% | 64% | 47% | 19% |
| C4F10/air (35:65) | 100% (3.2 x 10⁸/ml) | 97% | 79% | 64% | 17% |
| C3F8/air (65:35) | 100% (4.0 x 10⁸/ml) | 88% | 73% | 49% | 9% |

### Example 5

Lyophilisates were prepared as described in Example 3 using 25mg of DPPC, 25mg of DPPG (instead of DPPC, DPPA and DPPE-PEG5000). Aliquots of the tert-butanolic solution (500µl/vial) were frozen and lyophilized. The lyophilisate-containing vials were evacuated and filled with various gases (see below) under different reduced gas pressures (100, 300, 500, 700, 1000 mbar) and sealed with gas tight stoppers. The lyophilized samples were reconstituted with 5ml saline and Coulter analysis was performed. The results are presented in Table 5.

**Table 5**

| **Bubble conc. (%)** | | | | | |
|---|---|---|---|---|---|
| Gases | *(1000mbar)* | *(700mbar)* | *(500mbar)* | *(300mbar)* | *(100mbar)* |
| Air | 100% (0.46 x 10⁸/ml) | 25% | 20% | 9% | 0% |
| SF6 | 100% (1.8x 10⁸/ml) | - | 54% | 48% | 9% |
| C2F6 | 100% (4.7 x 10⁸/ml) | 40% | 39% | 27% | 8% |
| C4F10 | 100% (3.1 x 10⁸/ml) | 89% | 58% | 50% | 8% |
| C2F6/C4F10 (90:10) | 100% (3.3 x 10⁸/ml) | 72% | 59% | 42% | 13% |
| C4F10/air) (35:65 | 100% (2.8 x 10⁸/ml) | 52% | 56% | 54% | 11% |
| C3F8/air (65:35) | 100% (2.8 x 10⁸/ml) | 80% | 66% | 49% | 23% |

### Example 6

Lyophilisates were prepared as described in Example 3 using 47.5mg of DAPC, 2.5mg of DPPG (instead of DPPC, DPPA and DPPE-PEG5000). Aliquots of the tert-butanolic solution (250µl/vial) were frozen and lyophilized (instead of 310µl/vial in Example 3). The results of Coulter analysis are presented in Table 6 for each gas or gas mixture tested.

**Table 6**

| **Bubble conc. (%)** | | | | | |
|---|---|---|---|---|---|
| Gases | *(1000mbar)* | *(700mbar)* | *(500mbar)* | *(300mbar)* | *(100mbar)* |
| Air | 100% (0.8 x 10⁸/ml) | - | 62% | 23% | 0% |
| SF6 | 100% (4.7 x 10⁸/ml) | 70% | 70% | 40% | 1% |
| C2F6 | 100% (3.1 x 10⁸/ml) | 118% | 118% | 106% | 11% |
| C4F10 | 100% (3.2 x 10⁸/ml) | 128% | 148% | 90% | 23% |
| C2F6/C4F10 (90:10) | 100% (5.4 x 10⁸/ml) | 60% | 63% | 54% | 18% |
| C4F10/air (35:65) | 100% (3.6 x 10⁸/ml) | 108% | 115% | 74% | 15% |
| C3F8/air (65:35) | 100% (5.6 x 10⁸/ml) | 91% | 80% | 49% | 12% |

### Example 7

Lyophilisates were prepared as described in Example 6 with 22.5mg of DSPC, 22.5mg of DPPG and 5mg of palmitic acid (instead of DAPC, DPPG). The Coulter counter results are given in Table 7.

**Table 7**

| **Bubble conc. (%)** | | | | | |
|---|---|---|---|---|---|
| Gases | *(1000mbar)* | *(700mbar)* | *(500mbar)* | *(300mbar)* | *(100mbar)* |
| Air | 100% (1.2 x 10⁸/ml) | 49% | 22% | 11% | 0% |
| SF6 | 100% (2.3 x 10⁸/ml) | 106% | 48% | 22% | 16% |
| C2F6 | 100% (2.7 x 10⁸/ml) | 96% | 79% | 50% | 22% |
| C4F10 | 100% (3.1 x 10⁸/ml) | 99% | 96% | 58% | 29% |
| C2F6/C4F10 (90:10) | 100% (2.9 x 10⁸/ml) | 94% | 83% | 67% | 33% |
| C4F10/air (35:65) | 100% (2.7 x 10⁸/ml) | 109% | 85% | 64% | 29% |
| C3F8/air (65:35) | 100% (3.0 x 10⁸/ml) | 83% | 86% | 57% | 52% |

### Example 8

Lyophilisates were prepared as described in Example 6 with 25mg of DSPC, 22.5mg of DPPG and 2.5mg of Pluronic® F108 (instead of DAPC, DPPG). The Coulter counter results are given in Table 8.

**Table 8**

| **Bubble conc. (%)** | | | | | |
|---|---|---|---|---|---|
| Gases | *(1000mbar)* | *(700mbar)* | *(500mbar)* | *(300mbar)* | *(100mbar)* |
| Air | 100% (3.0 x 10⁸/ml) | 56% | 38% | 17% | 0% |
| SF6 | 100% (2.6 x 10⁸/ml) | 111% | 54% | 61% | 5% |
| C2F6 | 100% (4.4 x 10⁸/ml) | 92% | 66% | 69% | 15% |
| C4F10 | 100% (4.3 x 10⁸/ml) | 105% | 101% | 76% | 21% |
| C2F6/C4F10 (90:10) | 100% (4.7 x 10⁸/ml) | 89% | 90% | 67% | 28% |
| C4F10/air (35:65) | 100% (4.6 x 10⁸/ml) | 89% | 89% | 73% | 14% |
| C3F8/air (65:35) | 100% (5.1 x 10⁸/ml) | 85% | 86% | 70% | 24% |

Examples 3 to 8 show that the bubble formation under reduced gas pressures (<1000mbar) can be further improved or controlled by selecting the composition of the lipids and the gas or gas mixtures (solubility, molecular weight).

The following examples (Example 9-11) show that the use of a reduced gas pressure may help to control the microbubble size distribution, especially to eliminate large microbubbles (> 8µm). This is important to reduce the risk of gas embolism in blood vessels and to improve transpulmonary passage of microbubbles thus allowing reproducible and quantitative *in vivo* ultrasonic imaging properties.

### Example 9

Lyophilisates were prepared as described in Example 8 and filled with the gas C₄F₁₀ at various reduced gas pressures (see Table 9). The Coulter counter analyses were performed to determine bubble characteristics such as bubble concentration (Nb/ml), mean diameter in number (Dn) and in volume (Dv) and the bubble volume present in bubbles larger than 8µm (vol. > 8µm) expressed as a percentage of the bubble volume in bubbles larger than 8µm present at a pressure of 1000 mbar. The results are given in Table 9.

**Table 9**

| **Bubbles characteristics as a function of the gas pressure** | | | | | | |
|---|---|---|---|---|---|---|
| *Pressure in mbar* | *(1000)* | *(700)* | *(500)* | *(300)* | *(200)* | *(100)* |
| Nb/ml (x 10⁸) | 4.7 | 4.2 | 4.2 | 3.1 | 2.6 | 2.6 |
| Dn (µm) | 2.3 | 2.3 | 2.2 | 2.2 | 2.1 | 2.0 |
| Dv (µm) | 13.4 | 10.7 | 8.8 | 7.4 | 7.3 | 5.6 |
| Poly. Index * | 5.8 | 4.7 | 3.9 | 3.4 | 3.5 | 2.8 |
| Vol. > 8µm (%) | 100 | 58 | 35 | 18 | 10 | 4 |

| | | | | | | |
|---|---|---|---|---|---|---|
| * Polydispersity index = Dv/Dn | | | | | | |

This example clearly shows that without significantly changing the general bubble characteristics (Nb/ml, Dn), the use of a reduced pressure allows to control the bubble size distribution before reconstitution and especially to reduce considerably the number of large microbubbles (>8µm) that would not pass the lungs *in vivo* due to pulmonary filtration. Thus the number of bubbles produced under a pressure of 100 mbar is 55% of the value obtained at 1000 mbar (2.6x 10⁸ vs 4.7x 10⁸) but they contain 25 times less volume (Table 9 shows that the total volume in microbubbles larger than 8µm is reduced from 100% to 4%).

### Example 10

Lyophilisates were prepared as described in Example 8 and filled with a gas mixture of C₃F₈ and air (65:35 % by volume) at various reduced gas pressures. Coulter analyses were performed to determine the bubble characteristics such as the bubble concentration (Nb/ml), the mean diameter in number (Dn), the mean diameter in volume (Dv) and the bubble volume present in bubbles larger than 8µm as defined in example 9. The results are given in Table 10.

**Table 10**

| **Bubbles characteristics as a function of the gas pressure** | | | | | | |
|---|---|---|---|---|---|---|
| *Pressure in mbar* | *(1000)* | *(700)* | *(500)* | *(300)* | *(200)* | *(100)* |
| Nb/ml (x 10⁸) | 5.1 | 4.3 | 4.4 | 3.6 | 3.1 | 1.2 |
| Dn (µm) | 2.3 | 2.2 | 2.2 | 2.1 | 2.0 | 1.9 |
| Dv (µm) | 12.9 | 13.8 | 10.4 | 7.4 | 7.1 | 5.0 |
| Poly. Index | 5.7 | 6.2 | 4.8 | 3.5 | 3.6 | 2.7 |
| **Vol. > 8µm (%)** | **100** | **90** | **47** | **17** | **10** | **3** |

More than 50% of the of the volume present in large microbubbles (>8µm) is eliminated by using a reduced pressure of 500mbar when compared with the sample prepared at the atmospheric gas pressure (1000mbar). The other bubble properties remain substantially unchanged. The reduction is even more impressive at 200mbar where the volume in large (>8µm) bubbles falls to 10% of the value at atmospheric pressure whereas the total bubble concentration drops only by 39% (from 5.1 to 3.1x 10⁸/ml).

### Example 11

A phospholipid containing spray-dried formulation was obtained as follows:
0.45g of hydrogenated egg phosphophatidylcholine (Lipoid EPC-3) and 0.15g of Poloxamer 188 were dispersed in 100ml of distilled water. The dispersion was heated 1 hour at 50°C then cooled down to 4°C. 3ml of 1,1,2 trichlorotrifluoroethane (Freon® 113) was mixed with the dispersion using high speed homogenization (Polytron®, 2min at 10'000rpm and 4°C). The resulting emulsion was placed in a refrigerator 1 night then extruded through a Microfluidizer (Microfluidics Corp.) to reduce emulsion size (10'000psi, 5°C and 5 pass). An aqueous solution containing 3.6g of maltodextrin, 3g of NaCl and 2.6g of Na₂HPO₄ and 0.9g of NaH₂PO₄ was prepared in 100ml distilled water. This solution was mixed with the emulsion and then spray-dried (Mini Spray dryer Büchi 190: inlet air temperature, 235°C; outlet temperature, 105°C). The resulting dried powders were aliquoted into 10ml glass vials, filled with C₄F₁₀ under various gas pressures and then reconstituted with distilled water (30mg/ml). Coulter counter analyses were performed as before. The bubble characteristics obtained (Nb/ml, Dn, Dv, polydispersity index and the percentage of the bubble volume (> 8µm)) are summarized in Table 11.

**Table 11**

| **Bubbles characteristics as a function of the gas pressure** | | | | | |
|---|---|---|---|---|---|
| *Pressure in mbar* | *(1000)* | *(500)* | *(300)* | *(200)* | *(100)* |
| Nb/ml (x 10⁸) | 9.1 | 9.5 | 7.8 | 8.3 | 6.2 |
| Dn (µm) | 2.6 | 2.5 | 2.6 | 2.4 | 2.4 |
| Dv (µm) | 14.5 | 13.1 | 11.4 | 8.6 | 9.6 |
| Poly. Index * | 5.5 | 5.3 | 4.4 | 3.6 | 4.0 |
| **Vol. > 8µm (%)** | **100** | **71** | **46** | **26** | **17** |

| | | | | | |
|---|---|---|---|---|---|
| * Polydispersity index = Dv/Dn | | | | | |

Here again, the bubble size distribution is considerably narrowed by the use of a reduced gas pressure. This is especially true for the reduction in large microbubbles (>8µm). At 200mbar for instance, there are almost as many bubbles as at atmospheric pressure (8.3 vs 9.1x 10⁸/ml) but the volume of large bubbles (>8µm) is reduced by 74%. Thus the present invention allows improving the safety profile of this formulation.

### Example 12

### (Imaging in vivo)

This example shows the *in vivo* efficacy of the formulation of Example 11 (spray-dried phospholipid suspension) prepared under various reduced pressures. Briefly, overnight fasted minipigs were anaesthetized with Stressnil (Azaperone, Janssen: 2mg/kg) followed by Forene (Isofurane, Abbot). Left heart ventricle opacification was evaluated using an ATL HDI-5500 imaging system equipped with Pulse Inversion mode. The P4-2 probe (2.8/3.2 MHz) was used. Imaging dose (0.1ml/kg) and all setting parameters were optimized at the beginning of the experiment (acoustic power, gain, depth, focus, frame rates and processing) and kept constant for all tested samples. The results (peak intensity, duration and area under the intensity vs. time curve) are given in Table 12.

**Table 12**

| Tested sample | max. Intensity (PI) | Contrast duration (min) | Area under the curve |
|---|---|---|---|
| 1000mbar (ref.) | 85 | 5.3 | 6800 |
| 900mbar | 84 | 5.4 | 6810 |
| 500mbar | 80 | 5.4 | 6785 |
| 300mbar | 72 | 4.9 | 6110 |
| 200mbar | 81 | 4.6 | 5760 |
| 100mbar | 72 | 4.1 | 4300 |

The *in vivo* results show that even at 100mbar of the reduced gas pressure, the formulation is still highly effective. Similar results were obtained for contrast enhancement studies of the myocardium.

### Example 13

### (A contrast agent preparation from a kit)

The stability of gas microbubbles prepared under various reduced gas pressures was evaluated. Phospholipid containing lyophilisates as described in Example 7 were prepared in glass vials (DIN8R) and filled with 55% of C4F10 in air at 500 and 1000 mbar. A pre-filled saline syringe (containing 5ml of NaCl-0.9% solution) was connected to the vial for the reconstitution. For the 1000mbar sample (at 1 atmosphere), a Bio-Set® system equipped with a vent filter was used to compensate the overpressure resulting from the addition of the volume of saline into the vial; while for 500mbar sample, no vent was necessary. As the gas pressure in the vial is only half of the atmospheric pressure, when the syringe is connected to the vial, the vacuum (500mbar) automatically sucks in the saline solution until the liquid (5ml) is totally aspired. At this point, the gas pressure in the vial was restored to atmospheric pressure (1000mbar) by simple reconstitution. To compare the stability of microbubbles suspensions initially prepared under the normal (1000mbar) and reduced (500mbar) pressures, Coulter analyses were performed at t=0 and 6h after the reconstitution. The results (comparison of the bubble concentration and volume between t=0 and 6h after reconstitution) are summarized in Table 13.

**Table 13**

| | **Bubble conc. (Nb/ml)** | | **Bubble vol. (µl/ml)** | |
|---|---|---|---|---|
| | (t=0) | (t=6h) | (t=0) | (t=6h) |
| 1000mbar* | 2.6x 10⁸ | 2.6x 10⁸ | 17.7 | 13.7 |
| 500mbar* | 2.0x 10⁸ | 1.9x 10⁸ | 10.5 | 10.1 |

| | | | | |
|---|---|---|---|---|
| *Tested samples (n=5) | | | | |

The results show that the stability of the gas microbubbles suspensions prepared at 500mbar and 1000mbar are comparable.

### Example 14

### (Stability of the lyophilisate under reduced gas pressure during storage)

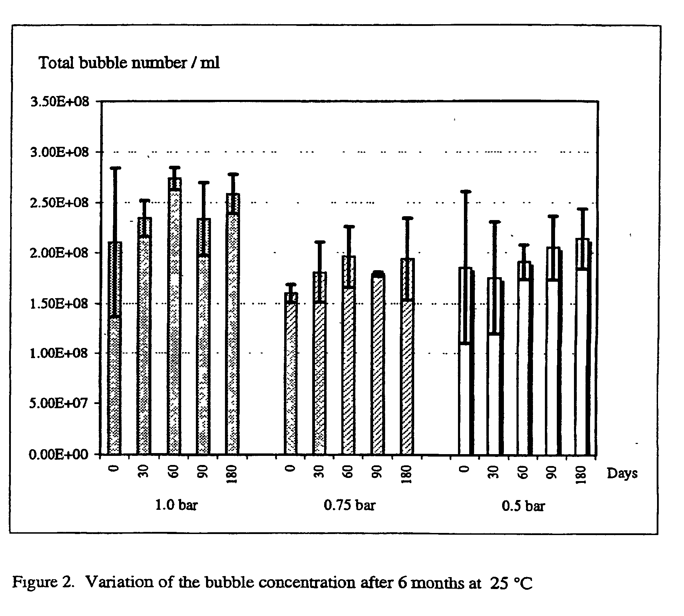

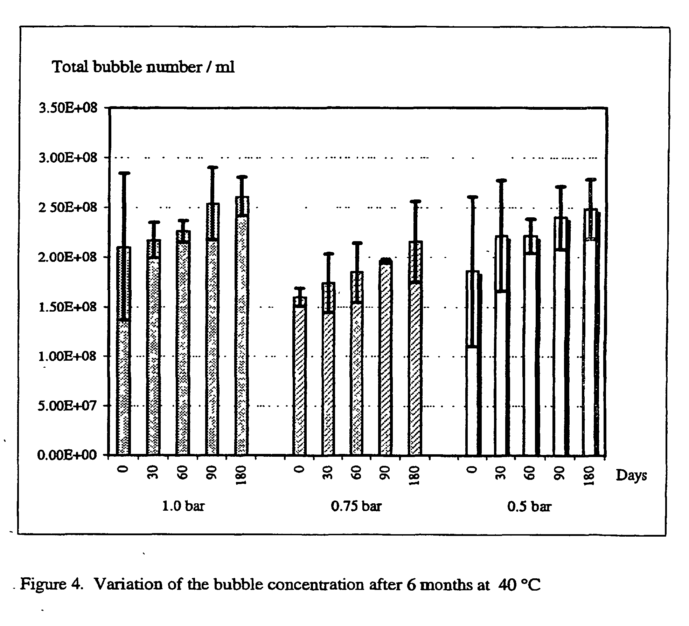

Phospholipid containing lyophilisates described in Example 7 were prepared in glass vials (DIN8R) and filled with 55% of C4F10 in air with gas pressures respectively at 500, 750 and 1000 mbar. The stability of lyophilized samples under the reduced gas pressures was evaluated after 0, 1, 3 and 6 months of storage at 25°C and 40°C. The Coulter analyses (bubble concentration) are given below (Table 14 and Figures 2 and 4).

**Table 14**

| **Stability studies at 25°C and 40°C (Nb/ml)** | | | | | |
|---|---|---|---|---|---|
| | **t=0** | **t=1 month** | | **t=3 months** | |
| | | 25°C | 40°C | 25°C | 40°C |
| 1000mbar* | 2.1x 10⁸ | 2.3x 10⁸ | 2.2x 10⁸ | 2.3x 10⁸ | 2.5x 10⁸ |
| 750mbar* | 1.6x 10⁸ | 1.8x 10⁸ | 1.7x 10⁸ | 1.8x 10⁸ | 2.0x 10⁸ |
| 500mbar* | 1.9x 10⁸ | 1.8x 10⁸ | 2.2x 10⁸ | 2.0x 10⁸ | 2.4x 10⁸ |

| | | | | | |
|---|---|---|---|---|---|
| *Tested samples (n=5) | | | | | |

The Coulter counter results showed no significant differences between the three lyophilized samples filled with different gas pressures after 1 and 3 months of storage.

The resistance to pressure was evaluated in vitro using a spectrophotometric method. The bubble suspension was diluted 1/50 in a solution of 0.9% sodium chloride and introduced in the sample cell of a spectrophotometer (Jenway 6100 Model). The cell was connected to a pneumatic valve allowing a linear pressure increase from 0 to 700 mmHg. The simultaneous recording of the pressure (via a pressure sensor) and of the absorbance provides typical curves allowing the determination of a "critical pressure" (Pc50) corresponding to the pressure at which the absorbance has decreased by 50%.

The resistances to pressure shown in Figures 3 and 5 are highly constant whatever the storage conditions. Temperature and pressure inside the vial have no influence on this parameter.
In conclusion, there is not a significant influence of the duration and the temperature of storage on the bubble characteristics (pressure resistance, bubble concentration).

### Example 15

Vials containing composition C were obtained as described in Example 2. After freeze drying, the vials were exposed to polarized helium gas (obtained from the Laue-Langevin Institute, Grenoble France) both under normal (1000mbar) and reduced gas pressures (500 and 100mbar). Bubble suspensions were obtained after injection of saline (1 ml) through the stoppers as described in example 2. The bubble suspensions (1 ml) were injected in the jugular vein of rats and imaged in a 2 Tesla MR imager with a 17 cm bore magnet and 6 cm diameter volumic coil tunable to both ¹H and ³He resonance frequency. The abdominal aorta was clearly visible. No significant differences between the vials with different gas pressures were detected.

## Claims

1. A contrast agent usable in diagnostic imaging comprising microbubbles of a gas or a gas mixture obtained by reconstitution in a physiologically acceptable liquid aqueous carrier of lyophilisable material obtainable by the process consisting of lyophilising lyophilisable material contained in a container said lyophilisable material comprising at least one phospholipid film-forming surfactant, effecting sealing closure of said container, **characterised in that**, prior to effecting sealing of said container, a vacuum is applied to said container and a reduced pressure comprised between 900 and 100 mbar of said gas or gas mixture is introduced into said container.

2. The contrast agent of claim 1 wherein the reduced pressure of said gas or gas mixture introduced into said container is comprised between 700 and 300 mbar.

3. The contrast agent of claim 1 wherein the reduced pressure of said gas or gas mixture introduced into said container is 500 mbar.

4. The contrast agent as claimed in any one of claims 1 to 3, in which the gas is selected from the group consisting of halogenated hydrocarbon gases, air, nitrogen, carbon dioxide, helium, krypton, xenon, argon, methane, hyperpolarized gases, hyperpolarized noble gases, radioactive gases sulphur hexafluoride and mixtures thereof.

5. The contrast agent according to claim 4, in which the gas is selected from the group consisting of fluorinated gases including sulphur hexafluoride, perfluorocarbons or mixtures thereof.

6. The contrast agent of claim 5, in which the perfluorocarbons are selected from the group consisting of perfluoroalkanes such as perfluoromethane, perfluoroethane, perfluoropropanes, perfluorobutanes, perfluoropentanes, perfluorohexanes, perfluoroheptanes; perfluoroalkenes such as perfluoropropene, perfluorobutenes, perfluorobutadiene; perfluoroalkynes such as perfluorobut-2-yne; perfluorocycloalkanes such as perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane, perfluorocycloheptane and mixtures thereof.

7. The contrast agent of claim 6, in which the perfluorocarbons are selected from the group consisting of perfluoromethane, perfluoroethane, perfluoropropane, perfluorobutane, perfluorocyclobutane, perfluoropentane, perfluorohexane and mixtures thereof.

8. The contrast agent according to claim 4, in which the hyperpolarized gas is selected from the group consisting of hyperpolarized helium, hyperpolarized xenon, hyperpolarized neon and mixtures thereof.

9. The contrast agent according to claim 4, in which the radioactive gases are selected from the group consisting of Xe¹³³ or Kr⁸¹ and mixtures thereof.

10. The contrast agent of any one of claims 1 to 9, wherein said phospholipid film-forming surfactant is a saturated phospholipid or a synthetic non-saturated phospholipid or mixtures thereof.

11. The contrast agent of claim 10, wherein the saturated phospholipid is selected from saturated phosphatidic acid, saturated phosphatidylcholine, saturated phosphatidyl-ethanolamine, saturated phosphatidyl-serine, saturated phosphatidylglycerol, saturated phosphatidyl-inositol, cardiolipin and sphingomyelin and mixtures thereof.

12. The contrast agent of claim 11, wherein the saturated phospholipid is selected from the group consisting of dimyristoylphosphatidylcholine, dipalmitoylphosphatidylcholine, distearoylphosphatidylcholine, diarachidoylphosphatidylcholine, dimyristoylphosphatidyl-serine, dipalmitoylphosphatidyl-serine, distearoylphosphatidyl-serine, diarachidoylphosphatidyl-serine, dimyristoylphosphatidylglycerol, dipalmitoylphosphatidylglycerol, distearoylphosphatidylgycerol, diarachidoylphosphatidylglycerol, dimyristoylphosphatidylethanolamine, dipalmitoylphosphatidylethanolamine, distearoylphosphatidylethanolamine, diarachidoylphosphatidylethanolamine, dimyristoylphosphatidic acid, dipalmitoylphosphatididic acid, distearoylphosphatididic acid, diarachidoylphosphatididic acid, dimyristoylphosphatidyl-inositol, dipalmitoylphosphatidyl-inositol, distearoylphosphatidyl-inositol or diarachidoylphosphatidyl-inositol and mixtures thereof.

13. The contrast agent of claim 1, wherein said lyophilised material further contains viscosity enhancers or stabilizers selected from linear and cross-linked poly- and oligo-saccharides, sugars, hydrophilic polymers like polyoxypropylene glycol and polyoxyethylene glycol.

14. The contrast agent of claim 1, wherein said lyophilised material further comprises up to 50% by weight of non phospholipid surfactants selected from fatty acids, esters and ethers of fatty acids and alcohols with polyols.

15. A sealed container comprising a lyophilised material and a gas or a gas mixture for use as contrast agent in diagnostic imaging, said lyophilised material comprising at least one phospholipid film-forming surfactant, **characterized in that** the gas or gas mixture contained in said sealed container has a reduced pressure comprised between 900 and 100 mbar.

16. The sealed container of claim 15, wherein the reduced pressure of said gas or gas mixture is comprised between 700 and 300 mbar.

17. The sealed container of claim 15 or 16, wherein the reduced pressure of said gas or gas mixture is preferably 500 mbar.

18. The sealed container as claimed in any one of claims 15 to 17, in which the gas is selected from the group consisting of halogenated hydrocarbon gases, air, nitrogen, carbon dioxide, helium, krypton, xenon, argon, methane, radioactive gases, hyperpolarized gases, sulphur hexafluoride and mixtures thereof.

19. The sealed container of any one of claims 15 to 18, wherein said phospholipid film-forming surfactant is a saturated phospholipid or a synthetic non-saturated phospholipid or mixtures thereof.

20. The sealed container of claim 19, wherein the saturated phospholipid is selected from phosphatidic acid, phosphatidylcholine, phosphatidyl-ethanolamine, phosphatidyl-serine, phosphatidylglycerol, phosphatidyl-inositol, cardiolipin, sphingomyelin and mixtures thereof.

21. A process for the preparation of a gas or a gas mixture containing lyophilised contrast agent usable in diagnostic imaging which comprises lyophilising lyophilisable material contained in a container said lyophilisable material comprising at least one phospholipid film-forming surfactant, effecting sealing closure of said container, **characterised in that**, prior to effecting sealing of said container, a vacuum is applied to said container and a reduced pressure comprised between 900 and 100 mbar of said gas or gas mixture is introduced into said container.

22. The process of claim 21, wherein the reduced pressure of said gas or gas mixture introduced into said container is preferably comprised between 700 and 300 mbar.

23. The process of claim 21 or 22, wherein the reduced pressure of said gas or gas mixture introduced into said container is more preferably 500 mbar.

24. The process as claimed in any one of claims 21 to 23, in which the gas or gas mixture is selected from the group consisting of halogenated hydrocarbon gases, air, nitrogen, carbon dioxide, helium, krypton, xenon, argon, methane, radioactive gases, hyperpolarized gases sulphur hexafluoride and mixtures thereof.

25. The process as claimed in any one of claims 21 to 24, wherein said phospholipid film-forming surfactant is a saturated phospholipid or a synthetic non-saturated phospholipid or mixtures thereof

26. The process of claim 25, wherein the saturated phospholipid is selected from phosphatidic acid, phosphatidylcholine, phosphatidyl-ethanolamine, phosphatidyl-serine, phosphatidylglycerol, phosphatidyl-inositol, cardiolipin, sphingomyelin and mixtures thereof.

27. The process of claim 21, wherein said lyophilised material further contains substances selected from dicetylphosphate, cholesterol, ergosterol, phytosterol, sitosterol, lanosterol, tocopherol, propyl gallate, ascorbyl palmitate and butylated hydroxy-toluene.

28. The process of claim 21, wherein said lyophilised material further contains viscosity enhancers or stabilizers selected from linear and cross-linked poly- and oligo-saecharides sugars, hydrophilic polymers like polyoxypropylene glycol and polyoxyethylene glycol.

29. The process of claim 21, wherein said lyophilised material further comprises up to 50% by weight of non phospholipid surfactants selected from fatty acids, esters and ethers of fatty acids and alcohols with polyols.

30. A process for the preparation of an injectable reconstituted suspension of air or gas filled microbubbles usable as imaging contrast agent in diagnostic imaging, **characterised in that** the content of the container as claimed in any one of claims 15 to 20 is admixed with water or a physiologically acceptable aqueous carrier liquid.

31. The use of the sealed container as claimed in any one of claims 15 to 20 for the manufacture of an injectable aqueous suspension of gas filled microbubbles by reconstitution of the content thereof in a physiologically acceptable aqueous carrier, for use in diagnosis involving diagnostic ultrasound imaging, nuclear medicine or in magnetic resonance imaging.

32. A two component kit comprising, as the first component, a lyophilised material and a gas or a gas mixture contained in a container as claimed in any one of claims 15 to 20 and, as the second component a physiologically acceptable carrier liquid which, when admixed with the first component, provides an injectable ultrasound or magnetic resonance contrast agent.

33. The contrast agent according to claim 1 to 14 for use in ultrasonic echography.

34. The contrast agent according to claim 8 for use in magnetic resonance imaging.

35. The contrast agent according to claim 9 for use in nuclear medicine.

## Patentansprüche

1. Kontrastmittel, das bei bildgebenden, diagnostischen Verfahren brauchbar ist, das Mikrobläschen eines Gases oder einer Gasmischung enthält, das durch Verdünnen eines lyophilisierbaren Materials in einem physiologisch verträglichen, flüssigen, wäßrigen Träger erhalten wurde, welches durch ein Verfahren erhältlich ist, bei dem man ein lyophilisierbares Material, das in einem Behälter enthalten ist, lyophilisiert, wobei das lyophilisierbare Material mindestens ein filmbildendes Phospholipidtensid enthält, man den Behälter dicht verschließt, **dadurch gekennzeichnet, daß** man den Behälter vor dem Verschließen unter Vakuum setzt und man anschließend das Gas oder die Gasmischung unter einem verminderten Druck zwischen 900 und 100 mbar in den Behälter einbringt.

2. Kontrastmittel nach Anspruch 1, bei dem der reduzierte Druck des in den Behälter eingebrachten Gases oder der Gasmischung zwischen 700 und 300 mbar liegt.

3. Kontrastmittel nach Anspruch 1, bei dem der reduzierte Druck des in den Behälter eingebrachten Gases oder der Gasmischung 500 mbar beträgt.

4. Kontrastmittel wie in einem der Ansprüche 1 bis 3 beansprucht, bei dem das Gas aus der aus der Gruppe ausgewählt ist, die aus halogenierten Kohlenwasserstoffgasen, Luft, Stickstoff, Kohlendioxid, Helium, Krypton, Xenon, Argon, Methan, hyperpolarisierten Gasen, hyperpolarisierten Edelgasen, radioaktiven Gasen, Schwefelfluorid und Mischungen davon besteht.

5. Kontrastmittel nach Anspruch 4, bei dem das Gas aus der Gruppe ausgewählt ist, die aus fluorierten Gasen besteht, einschließlich Schwefelhexafluorid, Perfluorkohlenstoffen oder Mischungen davon.

6. Kontrastmittel nach Anspruch 5, bei dem die Perfluorkohlenstoffe aus der Gruppe ausgewählt sind, die aus Perfluoralkanen, wie Perfluormethan, Perfluorethan, Perfluorpropanen, Perfluorbutanen, Perfluorpentanen, Perfluorhexanen, Perfluorheptanen; Perfluoralkenen, wie Perfluorpropen, Perfluorbutenen, Perfluorbutadien, Perfluoralkinen, wie Perfluorbut-2-in; Perfluorcycloalkanen, wie Perfluorcyclobutan, Perfluormethylcyclobutan, Perfluordimethylcyclobutanen, Perfluartrimethylcyclobutanen, Perfluorcyclopentan, Perfluormethylcyclopentan, Perfluordimethylcyclopentanen, Perfluorcyclohexan, Perfluormethylcyclohexan, Perfluorcycloheptan und Mischungen davon besteht.

7. Kontrastmittel nach Anspruch 6, bei dem die Perfluorkohlenstoffe aus der Gruppe ausgewählt sind, die aus Perfluormethan, Perfluorethan, Perfluorpropan, Perfluorbutan, Perfluorcyclobutan, Perfluorpentan, Perfluorhexan und Mischungen davon besteht.

8. Kontrastmittel nach Anspruch 4, bei dem das hyperpolarisierte Gas aus der Gruppe ausgewählt ist, die aus hyperpolarisiertem Helium, hyperpolarisiertem Xenon, hyperpolarisiertem Neon und Mischungen davon besteht.

9. Kontrastmittel nach Anspruch 4, bei dem die radioaktiven Gase aus der Gruppe ausgewählt sind, die aus Xe¹³³ oder Kr^{B1} und Mischungen davon besteht.

10. Kontrastmittel nach einem der Ansprüche 1 bis 9, bei dem das filmbildende Phospholipidtensid ein gesättigtes Phospholipid oder ein synthetisches, ungesättigtes Phospholipid oder eine Mischung davon ist.

11. Kontrastmittel nach Anspruch 10, bei dem das gesättigte Phospholipid aus gesättigter Phosphatidsäure, gesättigtem Phosphatidylcholin, gesättigtem Phosphatidylethanolamin, gesättigtem Phosphatidylserin, gesättigtem Phosphatidylglycerin, gesättigtem Phosphadilylinositol, Cardiolipin und Sphingomyelin und Mischungen davon ausgewählt ist.

12. Kontrastmittel nach Anspruch 11, bei dem das gesättigte Phospholipid aus der Gruppe ausgewählt ist, die aus Dimyristoylphosphatidylcholin, Dipalmitoylphosphatidylcholin, Distearoylphosphatidylcholin, Diarachidoylphosphatidylcholin, Dimyristoylphosphatidylserin, Dipalmitoylphosphatidylserin, Distearoylphosphatidylserin, Diarachidoylphosphatidylserin, Dimyristoylphosphatidylglycerin, Dipalmitoylphosphatidylglycerin, Distearoylphosphatidylglycerin, Diarachidoylphoaphatidylglycerin, Dimyristoylphoaphatidylethanolamin, Dipalmitoylphosphatidylethanolamin, Distearoylphosphatidylethanolamin, Diarachidoylphosphatidylethanolamin, Dimyristoylphosphatidsäure, Dipalmitoylphosphatidsäure, Distearoylphosphatidsäure, Diarachidoylphosphatidsäure, Dimyristoylphosphatidylinositol, Dipalmitoylphoaphatidylinositol, Distearoylphosphatidylinositol oder Diarachidoylphosphatidylinositol und Mischungen davon besteht.

13. Kontrastmittel nach Anspruch 1, bei dem das lyophilisierte Material weiter Viskositätsverstärker oder Stabilisatoren enthält, die aus linearem und vernetztem Poly- und Oligosacchariden, Zuckern, hydrophilen Polymeren, wie Polyoxypropylenglycol und Polyoxyethylenglycol, ausgewählt sind.

14. Kontrastmittel nach Anspruch 1, bei dem das lyophilisierte Material weiter bis zu 50 Gew.-% an nicht-phospholipid-Tensiden enthält, die aus Fettsäuren, Estern und Ethern von Fettsäuren und Alkoholen mit Polyolen ausgewählt sind.

15. Abgedichteter Behälter, der ein lyophilisiertes Material und ein Gas oder eine Gasmischung zur Verwendung als Kontrastmittel bei der bildgebenden, diagnostischen Untersuchung enthält, wobei das lyophilisierte Material mindestens ein filmbildendes Phospholipidtensid enthält, **dadurch gekennzeichnet, daß** das Gas oder die Gasmischung, die in dem abgedichteten Behälter enthalten ist, einen reduzierten Druck zwischen 900 und 100 mbar hat.

16. Abgedichteter Behälter nach Anspruch 15, in dem der reduzierte Druck des Gases oder der Gasmischung zwischen 700 und 300 mbar liegt.

17. Abgedichteter Behälter nach Anspruch 15 oder 16, in dem der reduzierte Druck des Gases oder der Gasmischung vorzugsweise 500 mbar ist.

18. Abgedichteter Behälter wie in einem der Ansprüche 15 bis 17 beansprucht, bei dem das Gas aus der Gruppe ausgewählt ist, die aus halogenierten Kohlenwasserstoffgasen, Luft, Stickstoff, Kohlendioxid, Helium, Krypton, Xenon, Argon, Methan, radioaktiven Gasen, hyperpolarisierten Gasen, Schwefelfluorid und Mischungen davon besteht.

19. Abgedichteter Behälter nach einem der Ansprüche 15 bis 18, bei dem das filmbildende Phospholipidtensid ein gesättigtes Phospholipid oder ein synthetisches, ungesättigtes Phospholipid oder eine Mischung davon ist.

20. Abgedichteter Behälter nach Anspruch 19, bei dem das gesättigte Phospholipid aus Phosphatidsäure, Phosphatidylcholin, Phosphatidylethanolamin, Phosphatidylserin, Phosphatidylglycerin, Phosphatidylinositol, Cardiolipin, Sphingomyelin und Mischungen davon ausgewählt ist.

21. Verfahren zur Herstellung eines lyophilisierten Kontrastmittels, das ein Gas oder eine Gasmischung enthält und bei der diagnostischen, bildgebenden Untersuchung brauchbar ist, bei dem man lyophilisierbares Material, das in einem Behälter enthalten ist, lyophilisiert, wobei das lyophilisierbare Material mindestens ein filmbildendes Phospholipidtensid enthält, man den Behälter dicht verschließt, **dadurch gekennzeichnet, daß** man vor dem Verschließen des Behälters den Behälter unter Vakuum setzt und man das Gas oder die Gasmischung mit einem reduzierten Druck zwischen 900 und 100 mbar in den Behälter einbringt.

22. Verfahren nach Anspruch 21, bei dem der reduzierte Druck des in den Behälter eingebrachten Gases oder der Gasmischung vorzugsweise zwischen 700 und 300 mbar liegt.

23. Verfahren nach Anspruch 21 oder 22, bei dem der reduzierte Druck des in den Behälter eingebrachten Gases oder der Gasmischung besonders bevorzugt 500 mbar beträgt.

24. Verfahren wie in einem der Ansprüche 21 bis 23 beansprucht, bei dem das Gas oder die Gasmischung aus der Gruppe ausgewählt ist, die aus halogenierten Kohlenwasserstoffgasen, Luft, Stickstoff, Kohlendioxid, Helium, Krypton, Xenon, Argon, Methan, radioaktiven Gasen, hyperpolarisierten Gasen, Schwefelfluorid und Mischungen davon besteht.

25. Verfahren wie in einem der Ansprüche 21 bis 24 beansprucht, bei dem das filmbildende Phospholipidtensid ein gesättigtes Phospholipid oder ein synthetisches, ungesättigtes Phospholipid oder eine Mischung davon ist.

26. Verfahren nach Anspruch 25, bei dem das gesättigte Phospholipid aus Phosphatidsäure, Phosphatidylcholin, Phosphatidylethanolamin, Phosphatidylserin, Phosphatidylglycerin, Phosphatidylinositol, Cardiolipin, Sphingomyelin und Mischungen davon ausgewählt ist.

27. Verfahren nach Anspruch 21, bei dem das gefriergetrocknete Material weiter Substanzen enthält, die aus Dicethylphosphat, Cholesterin, Ergosterin, Phytosterin, Sitosterin, Lanosterin, Tocopherol, Propylgallat, Ascorbylpalmitat und butyliertem Hydroxytoluol ausgewählt sind.

28. Verfahren nach Anspruch 21, bei dem das gefriergetrocknete Material weiter einen Viskositätsverstärker oder Stabilisatoren enthält, die aus linearen oder vernetzten Poly- und Oligosacchariden, Zuckern, hydrophilen Polymeren, wie Polyoxypropylenglycol und Polyoxiethylenglycol, ausgewählt sind.

29. Verfahren nach Anspruch 21, bei dem das gefriergetrocknete Material weiter bis zu 50 Gew.-% an nicht-phospholipid-Tensiden enthält, die aus Fettsäuren, Estern und Ethern von Fettsäuren und Alkoholen mit Polyolen ausgewählt sind.

30. Verfahren zur Herstellung einer injizierbaren, wiederhergestellten Suspension von luft- oder gasgefüllten Mikrobläschen, die als bildgebendes Kontrastmittel bei der bildgebenden, diagnostischen Untersuchung geeignet ist, **dadurch gekennzeichnet, daß** der Inhalt des in einem der Ansprüche 15 bis 20 beanspruchten Behälters mit Wasser oder einer physiologisch verträglichen, wäßrigen Trägerflüssigkeit gemischt wird.

31. Verwendung eines abgedichteten Behälters, wie in einem der Ansprüche 15 bis 20 beansprucht, zur Herstellung einer injizierbaren, wäßrigen Suspension von gasgefüllten Mikrobläschen durch Verdünnen des Inhalts davon mit einem physiologisch verträglichen, wäßrigen Träger, zur Verwendung bei der Diagnose, die eine bildgebende, diagnostische Ultraschalluntersuchung umfaßt, in der Nuklearmedizin oder Magnetresonanzbildgebung.

32. Zweikomponentenkit, der als erste Komponente ein gefriergetrocknetes Material und ein Gas oder eine Gasmischung, die in einem Behälter enthalten sind, wie in einem der Ansprüche 15 bis 20 beansprucht, und als zweite Komponente eine physiologisch verträgliche Trägerflüssigkeit enthält, die beim Mischen mit der ersten Komponente ein injizierbares Ultraschallkontrastmittel oder Kontrastmittel für die Magnetresonanzbildgebung ergibt.

33. Kontrastmittels gemäß Anspruch 1 bis 14 zur Verwendung in der Ultraschallechographie.

34. Kontrastmittels gemäß Anspruch 8 zur Verwendung in der Magnetreaonanzbildgebung.

35. Kontrastmittels gemäß Anspruch 9 zur Verwendung in der Nuklearmedizin.

## Revendications

1. Agent de contraste utilisable dans l'imagerie de diagnostic comprenant des microbulles d'un gaz ou d'un mélange de gaz obtenues par reconstitution dans un transporteur aqueux liquide physiologiquement acceptable d'un matériau lyophilisable que l'on peut obtenir par le processus consistant à lyophiliser le matériau lyophilisable contenu dans un récipient, ledit matériau lyophilisable comprenant au moins un tensioactif filmogène phospholipidique, à effectuer la fermeture par scellement dudit récipient, **caractérisé en ce que**, avant d'effectuer le scellement dudit récipient, un vide est appliqué audit récipient et une pression réduite comprise entre 900 et 100 mbars dudit gaz ou mélange de gaz est introduite dans ledit récipient.

2. Agent de contraste selon la revendication 1, dans lequel la pression réduite dudit gaz ou mélange de gaz introduite dans ledit récipient est comprise entre 700 et 300 mbars.

3. Agent de contraste selon la revendication 1, dans lequel la pression réduite dudit gaz ou mélange de gaz introduite dans ledit récipient est de 500 mbars.

4. Agent de contraste selon l'une quelconque des revendications 1 à 3, dans lequel le gaz est choisi dans le groupe constitué des gaz d'hydrocarbure halogéné, de l'air, de l'azote, du dioxyde de carbone, de l'hélium, du krypton, du xénon, de l'argon, du méthane, des gaz hyperpolarisés, des gaz nobles hyperpolarisés, des gaz radioactifs, du fluorure de soufre et de leurs mélanges.

5. Agent de contraste selon la revendication 4, dans lequel le gaz est choisi dans le groupe constitué des gaz fluorés comprenant l'hexafluorure de soufre, les perfluorocarbones ou leurs mélanges.

6. Agent de contraste selon la revendication 5, dans lequel les perfluorocarbones sont choisis dans le groupe constitué des perfluoroalcanes tels que le perfluorométhane, le perfluoroéthane, les perfluoropropanes, les perfluorobutanes, les perfluoropentanes, les perfluorohexanes, les perfluoroheptanes; les perfluomalcènes tels que le perfluoropropène, les perlfuorobutènes, le perlfuorobutadiène; les perfluoroalcynes tels que la perfluorobut-2-yne ; les perfluorocycloalcanes tels que le perfluorocyclobutane, le perfluorométhylcyclobutane, les perfluorodiméthylcyclobutanes, les perfluorotriméthylcyclobutanes, le perlfuorocyclopentane, le perlfuorométhylcyclopentane, les perfluorodiméthylcyclopentanes, le perfluorocyclohexane, le perfluorométhylcyclohexane, le perfluorocycloheptane et leurs mélanges.

7. Agent de contraste selon la revendication 6, dans lequel les perfluorocarbones sont choisis dans le groupe constitué du perfluorométhane, du perfluoroéthane, du perfluoropropane, du perfluorobutane, du perfluorocyclobutane, du perfluoropentane, du perfluorohexane et de leurs mélanges.

8. Agent de contraste selon la revendication 4, dans lequel le gaz hyperpolarisé est choisi dans le groupe constitué de l'hélium hyperpolarisé, du xénon hyperpolarisé, du néon hyperpolarisé et de leurs mélanges.

9. Agent de contraste selon la revendication 4, dans lequel les gaz radioactifs sont choisis dans le groupe constitué du Xe¹³³ ou du Kr⁸¹ et de leurs mélanges.

10. Agent de contraste selon l'une quelconque des revendications 1 à 9, dans lequel ledit tensioactif filmogène phospholipidique est un phospholipide saturé ou un phospholipide non saturé synthétique ou leurs mélanges.

11. Agent de contraste selon la revendication 10, dans lequel le phospholipide saturé est choisi parmi l'acide phosphatidique saturé, la phosphatidylcholine saturée, la phosphatidyl-éthanolamine saturée, la phosphatidyl-sérine saturée, le phosphatidylglycérol saturé, le phosphatidylinositol saturé, la cardiolipine et la sphingomyéline et leurs mélanges.

12. Agent de contraste selon la revendication 11, dans lequel le phospholipide saturé est choisi dans le groupe constitué de la dimyristoylphosphatidylcholine, la dipalmitoylphosphatidylcholine, la distéaroylphosphatidylcholine, la diarachidoylphosphatidylcholine, la dimyristoylphosphatidyl-sérine, la dipalmitoylphosphatidyl-sérine, la distéaroylphosphatidyl-sérine, la diarachidoylphosphatidyl-sérine, le dimyristoylphosphatidylglycérol, le dipalmitoylphosphatidylglycérol, le distéaroylphosphatidylglycérol, le diarachidoylphosphatidylglycérol, la dimyristoylphosphatidyléthanolamine, la dipalmitoylphosphatidyléthanolamine, la distéaroylphosphatidyléthanolamine, la diarachidoylphosphatidyléthanolamine, l'acide dimyristoylphosphatidique, l'acide dipalmitoylphosphatidique, l'acide distéaroylphosphatidique, l'acide diarachidoylphosphatidique, le dimyristoylphosphatidyl-inositol, le dipalmitoylphosphatidyl-inositol, le distéaroylphosphatidyl-inositol ou le diarachidoylphosphatidyl-inositol et leurs mélanges.

13. Agent de contraste selon la revendication 1, dans lequel ledit matériau lyophilisé contient en outre des amplificateurs de viscosité ou des stabilisants choisis parmi les poly- et oligo-saccharides linéaires et réticulés, les sucres, les polymères hydrophiles comme le polyoxypropylène glycol et le polyoxyéthylène glycol.

14. Agent de contraste selon la revendication 1, dans lequel ledit matériau lyophilisé comprend en outre jusqu'à 50 % en poids de tensioactifs non phospholipidiques choisis parmi les acides gras, les esters et les éthers d'acides gras et les alcools avec des polyols.

15. Récipient scellé comprenant un matériau lyophilisé et un gaz ou un mélange de gaz pour une utilisation comme agent de contraste dans l'imagerie de diagnostic, ledit matériau lyophilisé comprenant au moins un tensioactif filmogène phospholipidique, **caractérisé en ce que** le gaz ou mélange de gaz contenu dans ledit récipient scellé a une pression réduite comprise entre 900 et 100 mbars.

16. Conteneur scellé selon la revendication 15, dans lequel la pression réduite dudit gaz ou mélange de gaz est comprise entre 700 et 300 mbars.

17. Récipient scellé selon la revendication 15 ou 16, dans lequel la pression réduite dudit gaz ou mélange de gaz est de préférence de 500 mbars.

18. Récipient scellé selon l'une quelconque des revendications 15 à 17, dans lequel le gaz est choisi dans le groupe constitué des gaz d'hydrocarbure halogéné, de l'air, de l'azote, du dioxyde de carbone, de l'hélium, du krypton, du xénon, de l'argon, du méthane, des gaz radioactifs, des gaz hyperpolarisés, du fluorure de soufre et de leurs mélanges.

19. Récipient scellé selon l'une quelconque des revendications 15 à 18, dans lequel ledit tensioactif filmogène phospholipidique est un phospholipide saturé ou un phospholipide non saturé synthétique ou leurs mélanges.

20. Récipient scellé selon la revendication 19, dans lequel le phospholipide saturé est choisi parmi l'acide phosphatidique, la phosphatidylcholine, la phosphatidyl-éthanolamine, la phosphatidyl-sérine, le phosphatidylglycérol, le phosphatidyl-inositol, la cardiolipine, la sphingomyéline et leurs mélanges.

21. Processus pour la préparation d'un gaz ou d'un mélange de gaz contenant un agent de contraste lyophilisé utilisable dans l'imagerie de diagnostic comprenant la lyophilisation d'un matériau lyophilisable contenu dans un récipient, ledit matériau lyophilisable comprenant au moins un tensioactif filmogène phospholipidique, la fermeture par scellement dudit récipient, **caractérisé en ce que**, avant d'effectuer le scellement dudit récipient, un vide est appliqué audit récipient et une pression réduite comprise entre 900 et 100 mbars dudit gaz ou mélange de gaz est introduite dans ledit récipient.

22. Processus selon la revendication 21, dans lequel la pression réduite dudit gaz ou mélange de gaz introduite dans ledit récipient est comprise de préférence entre 700 et 300 mbars.

23. Processus selon la revendication 21 ou 22, dans lequel la pression réduite dudit gaz ou mélange de gaz introduite dans ledit récipient est de manière davantage préférée de 500 mbars.

24. Processus selon l'une quelconque des revendications 21 à 23, dans lequel le gaz ou mélange de gaz est choisi dans le groupe constitué des gaz d'hydrocarbure halogéné, de l'air, de l'azote, du dioxyde de carbone, de l'hélium, du krypton, du xénon, de l'argon, du méthane, des gaz radioactifs, des gaz hyperpolarisés, du fluorure de soufre et de leurs mélanges.

25. Processus selon l'une quelconque des revendications 21 à 24, dans lequel ledit tensioactif filmogène phospholipidique est un phospholipide saturé ou un phospholipide non saturé synthétique ou leurs mélanges.

26. Processus selon la revendication 25, dans lequel le phospholipide saturé est choisi parmi l'acide phosphatidique, la phosphatidylcholine, la phosphatidyl-éthanolamine, la phosphatidyl-sérine, le phosphatidylgiycérol, le phosphatidyl-inositol, la cardiolipine, la sphingomyéline et leurs mélanges.

27. Processus selon la revendication 21, dans lequel ledit matériau lyophilisé contient en outre des substances choisies parmi le dicétylphosphate, le cholestérol, l'ergostérol, le phytostérol, le sitostérol, le lanostéral, le tocophérol, le gallate de propyle, le palmitate d'ascorbyle et l'hydroxy-toluène butylé.

28. Processus selon la revendication 21, dans lequel ledit matériau lyophilisé contient en outre des amplificateurs de viscosité ou des stabilisants choisis parmi les poly- et oligo-saccharides linéaires et réticulés, les sucres, les polymères hydrophiles comme le polyoxypropylène glycol et le polyoxyéthylène glycol.

29. Processus selon la revendication 21, dans lequel ledit matériau lyophilisé comprend en outre jusqu'à 50 % en poids de tensioactifs non phospholipidiques choisis parmi les acides gras, les esters et les éthers d'acides gras et les alcools avec des polyols.

30. Processus pour la préparation d'une suspension reconstituée injectable de microbulles remplies d'air ou de gaz utilisable comme agent de contraste d'imagerie dans l'imagerie de diagnostic, **caractérisé en ce que** le contenu du récipient selon l'une quelconque des revendications 15 à 20 est mélangé avec de l'eau ou un liquide transporteur aqueux physiologiquement acceptable.

31. Utilisation du récipient scellé selon l'une quelconque des revendications 15 à 20, pour la fabrication d'une suspension aqueuse injectable de microbulles remplies de gaz par reconstitution de son contenu dans un transporteur aqueux physiologiquement acceptable, pour une utilisation dans le diagnostic impliquant l'imagerie de diagnostic par ultrasons, la médecine nucléaire ou dans l'imagerie par résonance magnétique.

32. Kit à deux composants comprenant, comme premier composant, un matériau lyophilisé et un gaz ou un mélange de gaz contenus dans un récipient selon l'une quelconque des revendications 15 à 20 et, comme second composant, un liquide transporteur physiologiquement acceptable qui, quand il est mélangé avec le premier composant, donne un agent de contraste d'ultrasons ou de résonance magnétique injectable.

33. Agent de contraste selon la revendication 1 à 14, pour utilisation en échographie par ultrasons.

34. Agent de contraste selon la revendication 8, pour utilisation en limagerie par résonance magnétique.

35. Agent de contraste selon la revendication 9, pour utilisation en médecine nucléaire.
